# EUROPEAN PATENT APPLICATION

(11) **EP 1 621 612 A1**
(43) Date of publication of application: **01.02.2006**
(21) Application number: 04724621.0
(22) Date of filing: 31.03.2004
(51) Int. Cl.: C12N 7/04, C12N 15/866, A61K 39/12

(54) **COMPLETE EMPTY VIRAL PARTICLES OF INFECTIOUS BURSAL DISEASE VIRUS (IBDV), PRODUCTION METHOD THEREOF AND APPLICATIONS OF SAME**

(30) Priority: 31.03.2003 ES 200300751 P
(71) Applicant: CONSEJO SUPERIOR DE INVESTIGACIONES CIENTIFICAS, 28006 Madrid (ES); Bionostra S.L., 28760 Tres Cantos (ES)
(72) Inventor: RODRIGUEZ AGUIRRE, José Francisco, 28006 Madrid (ES); GONZALES DE LLANO, Dolores, 28006 Madrid (ES); ONA BLANCO, Ana Maria, 28006 Madrid (ES); ABAITUA ELUSTONDO, Fernando, 28006 Madrid (ES); MARAVER MOLINA, Antonio, 28006 Madrid (ES); CLEMENTE CERVERA, Roberto, 28006 Madrid (ES); RUIZ CASTON, José, 28006 Madrid (ES); RODRIGUEZ FERNANDEZ-ALBA, Juan Ramon, 28006 Madrid (ES)
(74) Representative: ABG Patentes, S.L.
(86) International application number: PCT/ES2004/000147
(87) International publication number: WO 2004/087900

(57) **Abstract**

The whole empty viral particles of the infectious bursal disease virus (IBDV) inducer contain all the antigenically relevant protein components in the determining IBDV virions and can be obtained by means of genetic engineering in suitable expression systems. Said capsids are useful in the production of vaccines against the avian disease called infectious bursal disease caused by IBDV and in the manufacture of gene therapy vectors.

## Description

### FIELD OF THE INVENTION

The invention is related to whole empty viral particles of the infectious bursal disease virus (IBDV), with immunogenic activity against IBDV, their production by means of genetic engineering and applications thereof, particularly in the production of animal health vaccines, for example, in the manufacture of vaccines against the avian disease called infectious bursal disease caused by IBDV and in the manufacture of gene therapy vectors.

### BACKGROUND OF THE INVENTION

During the last four decades of the 20^{th} century, the appearance and global spreading of an avian disease called infectious bursal disease (IBD) occurred. IBD is characterized by the destruction of pre-B lymphocyte populations residing in the bursa of Fabricius of infected animals (Sharma JM et al. 2000. Infectious bursal disease virus of chickens: pathogenesis and immunosuppression. Dev Comp Immunol. 24:223-35). This disease is caused by the infectious bursal disease virus (IBDV) belonging to the *Birnaviridae* family (Leong JC et al. 2000. Virus Taxonomy Seventh Report of International Committee on Taxonomy of Viruses. Academic Press, San Diego, CA). In spite of the implementation of intensive vaccination programs, based on the use of combinations of live and inactivated vaccines, outbreaks of IBD are still reported in all chicken meat-producing countries (van den Berg TP et al. 2000. infectious bursal disease (Gumboro disease). Rev Sci Tech. 19:509-43).

The virions of the infectious bursal virus lack a lipid envelope, have an icosahedral structure (symmetry T-13) and have a diameter of 65-70 nm (Bottcher B. et al. 1997. Three-dimensional structure of infectious bursal disease virus determined by electron cryomicroscopy. J Virol. 71:325-30; Castón JR et al., 2001. C terminus of infectious bursal disease virus major capsid protein VP2 is involved in definition of the t number for capsid assembly. J Virol. 75:10815-28). The capsid is formed by a single protein layer containing four different polypeptides called VPX, VP2, VP3 and VP1, respectively. The VPX, VP2 and VP3 proteins are produced by means of proteolytic processing of a precursor, referred to as viral polyprotein, encoded by genomic segment A. The VP1. protein is produced by means of expression of the corresponding gene encoded by segment B.

The viral polyprotein, synthesized as a precursor of 109 kDa, is processed cotranslationally, giving rise to the formation of three polypeptides referred to as VPX, VP3 and VP4. VP4 is responsible for this processing (Birghan C. et al. 2000. A non-canonical Ion proteinase lacking the ATPase domain employs the Ser-Lys catalytic dyad to exercise broad control over the life cycle of a double-stranded RNA virus. Embo J. 19:114-23). VP3 is a polypeptide of 29 kDa forming trimeric subunits coating the inner layer of the capsid. VPX (also known as pVP2) undergoes a second proteolytic processing giving way to the mature form of the protein called VP2. The outer surface of the virions is formed by trimeric subunits constituted of a variable ratio of VPX and VP2 (Chevalier C et al. 2002. The maturation process of pVP2 requires assembly of infectious bursal disease virus capsids. J Virol. 76:2384-92; Lombardo E et al. 1999. VP1, the putative RNA-dependent RNA polymerase of infectious bursal disease virus, forms complexes with the capsid protein VP3, leading to efficient encapsidation into virus-like particles. J Virol. 73:6973-83). It has been suggested that the conversion of VPX to VP2 is associated with the formation of mature capsids (Chevalier C et al. 2002. The maturation process of pVP2 requires assembly of infectious bursal disease virus capsids. J Virol. 76:2384-92; Martínez-Torrecuadrada JL. 2000. Different architectures in the assembly of infectious bursal disease virus capsid proteins expressed in insect cells. Virology. 278:322-31). The polyprotein proteolytic processing sites have been characterized (Da Costa B et al. 2002. The capsid of infectious bursal disease virus contains several small peptides arising from the maturation process of pVP2. J Virol. 76:2393-402; Sánchez AB & Rodriguez JF. 1999. Proteolytic processing in infectious bursal disease virus: identification of the polyprotein cleavage sites by site-directed mutagenesis. Virology. 262:190-9), which allows for a reliable expression of the polypeptides of the capsid. The viral RNA-dependent RNA polymerase (RdRp) viral, called VP1, interacts with the VP3 protein, giving rise to a complex facilitating its encapsidation (Lombardo E ct al. 1999. VP1, the putative RNA-dependent RNA polymerase of infectious bursal disease virus, forms complexes with the capsid protein VP3, leading to efficient encapsidation into virus-like particles. J Virol. 73:6973-83; Tacken M et al. 2000. Interactions in vivo between the proteins of infectious bursal disease virus: capsid protein VP3 interacts with the RNA-dependent RNA polymerase, VP1. J Gen Virol. 81 Pt 1:209-18). The domain of the protein VP3 responsible for this interaction is located in its 16 C-terminal residues (Maraver A et al. Identification and molecular characterization of the RNA polymerase-binding motif of the inner capsid protein VP3 of infectious bursal disease virus. J. Virol. 77:2459-2468). The protein VP3 interacts with RNA unspecifically. This reaction does not require the existence of specific sequences in the RNA molecule (Kochan G et al. 2003. Characterization of the RNA binding activity of VP3, a major structural protein of IBDV. Archives of Virology 148:723-744). As with that observed with other internal capsid proteins of other viruses, it seems likely, that VP3 stabilizes the genomic RNA in the viral particle.

Conventional vaccines used for controlling infectious bursal disease are based on the use of strains, with different degrees of virulence, of the IBDV itself grown in cell culture or in embryonated eggs. The extracts containing the infectious material are subjected to chemical inactivation processes to produce inactivated vaccines, or else are used directly to produce live attenuated vaccines (Sharma JM et al. 2000. Infectious bursal disease virus of chickens: pathogenesis and immunosuppression. Developmental and Comparative Immunology 24:223-235; van den Berg TP et al. 2000. Rev Sci Tech 2000, 19:509-543). This latter type of vaccines has the typical drawbacks associated with the use of live attenuated vaccines, specifically, the risk of mutations reverting the virulence of the virus or making it lose its immunogenicity.

Recombinant subunit vaccines containing the IBDV protein VP2 expressed in several expression systems, for example, bacteria, yeasts or baculovirus, usually in fusion protein form, have been disclosed. The results obtained in chicken immunization tests with said vaccines have not been completely satisfactory.

Empty viral capsids or virus-like particles (VLPs,) constitute an alternative to the use of live attenuated vaccines and of recombinant subunit vaccines. VLPs are obtained by self-assembly of the subunits constituting the viral capsid and mimicking the structure and antigenic properties of the native virion, even thought they lack genetic material, as a result of which they are incapable of replicating themselves. Apart from their application for vaccination purposes, VLPs can be used as vectors of molecules of biological interest, for example, nucleic acids, peptides or proteins. By way of illustration, parvovirus VLPs (US 6,458,362) or human immunodeficiency virus (HIV) VLPs (US 6,602,705), can be mentioned.

Morphogenesis is a vital process for the viral cycle requiring successive steps associated to modifications in the polypeptide precursors. As a result, viruses have developed strategies allowing the sequential and correct interaction between each one of its components. One of these strategies, frequently used by icosahedral viruses, is the use of polypeptides coming from a single polyprotein as the base of its structural components. In these cases, the suitable proteolytic processing of said polyprotein plays a crucial role in the assembly process.

The production of several IBDV VLPs by means of expression of the viral polyprotein using different expression systems have been disclosed. In 1997, Vakharia disclosed for the first time, obtainment of IBDV VLPs in insect cells (Vakharia, V. N. 1997. Development of recombinant vaccines against infectious bursal disease. Biotechnology Annual Review 3:151-68). Later, in 1998, the research group to which the inventors belonged proved the possibility of obtaining IBDV VLPs in mammal cells (Fernández-Arias A et al. 1998. Expression of ORF A1 of infectious bursal disease virus results in the formation of virus-like particles, J. Gen. Virol. 79:1047-54). In 1999, an article was published disclosing the obtaining of IBDV VLPs in insect cells by another research group (Kibenge FS et al. 1999. Formation of virus-like particles when the polyprotein gene (segment A) of infectious bursal disease virus is expressed in insect cells. Can J Vet Res 63:49-55). A subsequent study, published by the laboratory to which the inventors belong, in collaboration with INGENASA S.A., proved that the morphogenesis of IBDV VLPs in insect cells infected with recombinant baculoviruses expressing the IBDV polyprotein is very ineffective and leads to the major accumulation of abnormal tubular structures (Martínez-Torrecuadrada JL et al. 2000. Different architectures in the assembly of infectious bursal disease virus capsid proteins expressed in insect cells. Virology 278:322-331). These results were subsequently corroborated (Chevalier C ct al. 2002. The maturation process of pVP2 requires assembly of infectious bursal disease virus capsids. J. Virol. 76:2384-92). In that same article, that group of researchers proved the possibility of obtaining an efficient morphogenesis by means of the expression of a chimeric polyprotein formed by the fusion of the open reading frame (ORF) corresponding to the green fluorescent protein (GFP) and to 3' end of the open reading phase of the IBDV polyprotein. The expression of this chimeric polyprotein leads to the formation of recombinant IBDV VLPs, containing in their interior a VP3-GFP recombinant fusion protein, different from the one present in the IBDV virions. On the other hand, the results disclosed in this latter research project do not provide information concerning the mechanism responsible for the ineffectiveness of the morphogenetic process of the IBDV VLPs in insect cells.

It is important to stress that all the VLPs disclosed previously lack the VP1 protein, which is present in the IBDV virions. The only reference to the obtaining of IBDV VLPs including VP1 have been carried out by researchers of the laboratory that the inventors belong to (Lombardo E et al. 1999. VP1, the putative RNA-dependent RNA polymerase of infectious bursal disease virus, forms complexes with the capsid protein VP3, leading to efficient encapsidation into virus-like particles. J. Virol. 73:6973-83), using the vaccine virus as the vector, which prevents the possible use of said VLPs for vaccination purposes.

The different processes of producing IBDV VLPs previously described suffer from different defects reducing or preventing their applicability for the generation of vaccines against IBDV, given that:
i) the production of IBDV VLPs in mammal cells is based on the use of recombinants of the vaccine virus; however, that production system has a very high cost and, as it uses a recombinant virus capable of infecting both mammals and birds, it does not meet the biosafety conditions necessary for its use as a vaccine;
ii) the production of IBDV VLPs in insect cells using conventional expression systems, i.e. recombinant baculoviruses only expressing the viral polyprotein, is very inefficient, leading to practically no production of VLPs;
iii) the production of IBDV VLPs in insect cells by means of the expression of a chimeric polyprotein (formed by the fusion of the ORF corresponding to the GFP at the 3' end of the ORF corresponding to the IBDV polyprotein) results in the production of IBDV VLPs containing a fusion protein VP3-GFP, which introduces a protein clement not present in IBDV virions, of unknown effect and of doubtful applicability in the chicken food chain for human consumption, and
iv) none of the systems described above for the production of IBDV VLPs based on the use of recombinant baculoviruses allows for obtaining IBDV VLPs containing all the antigens present in the IBDV virions.

### SUMMARY OF THE INVENTION

The invention generally is aimed at the problem of providing new effective and safe vaccines against the infectious bursal disease virus (IBDV).

The solution provided by this invention is based on it being possible to obtain IBDV VLPs correctly assembled by means of the simultaneous expression of the viral polyprotein and the IBDV VP1 protein from two independent open reading frames (ORFs) in suitable host cells. In a particular embodiment, the expression of said ORFs is controlled by different promoters. Said IBDV VLPs are formed by auto-assembly of the IBDV VPX, VP2, VP3 and VP1 proteins, whereby they contain all the antigenically relevant protein elements present in the purified and infective IBDV virions and, for this reason, are called "whole IBDV VLPs" in this description. Given that said whole (complete) IBDV VLPs contain all the antigenically relevant protein elements present in the purified and infective virions of IBDV so as to induce an immunogenic or antigenic response, said whole IBDV VLPs can be used for therapeutic purposes, for example, in the development of vaccines, such as vaccines for protecting birds from the infection caused by IBDV or in the development of gene therapy vectors; for diagnostic purposes, etc.

The obtained results clearly show that: (i) IBDV VP3 protein, expressed in insect cells from the expression of the viral polyprotein, undergo a proteolytic processing eliminating the last 13 amino acid residues from its C-terminal end; (ii) the resulting VP3 protein (called VP3T) is incapable of forming oligomers, which produces a virtually complete blocking of the morphogenetic process inducing virtually no production of VLPs; and (iii) the association of the VP3 protein with the VP1 protein protects the first one (VP3) against the proteolytic processing.

These results have allowed for designing a new strategy or process for the efficient production of whole IBDV VLPs and which, unlike the previously described methods, have an effective morphogenesis while at the same time the presence therein of heterologous protein elements inexistent in purified viral particles is prevented. This strategy is based on the use of a gene expression vector or system allowing the coexpression of the viral polyprotein and of the VP1 protein as independent ORFs, which assures the presence of the viral polyprotein and of the IBDV VP1 protein during the assembly process of the whole IBDV VLPs. Under these conditions, the VP3 and VP1 proteins form stable complexes hindering the proteolytic degradation of VP3, assuring its proper functioning, and leading to the incorporation of VP1 in the IBDV VLPs.

In a particular embodiment, said gene expression system is based on the use of a dual recombinant baculovirus simultaneously expressing the viral polyprotein and the IBDV VP1 protein from two independent ORFs controlled by different promoters. In another particular embodiment, said whole IBDV VLPs are obtained as a result of the coinfection of host cells, such as insect cells, with two recombinant baculoviruses, one of them capable to express the viral polyprotein and the other one, the IBDV VP1 protein.

The vaccines obtained by using said whole IBDV VLPs have a number of advantages since it prevents the handling of highly infectious material, it prevents the potential risk of the occurrence of new IBDV mutants, and eliminates the use of a live virus on poultry farms, thus preventing the risk of spreading IBDV vaccine strains to the environment.

Consequently, one aspect of the present invention is related to a whole IBDV VLP made up by assembly of the IBDV PVX, VP2, VP3 and VP1 proteins. Said whole IBDV VLP has antigenic or immunogenic activity against the infection caused by IBDV.

A further aspect of this invention is related to a process for the production of said whole IBDV VLPs provided by this invention, based on the gene coexpression of the viral polyprotein and of the IBDV VP1 as two independent ORFs in suitable host cells. In a particular embodiment, the expression of said ORFs is controlled by different promoters.

The gene constructs, expression systems and host cells developed for the implementation of said production process of said whole IBDV VLPs, as well as their use for the production of said whole IBDV VLPs, constitute further aspects of the present invention.

Said whole IBDV VLPs have the ability to immunize animals, particularly, birds, against the avian disease caused by IBDV, as well as the ability to vectorize or incorporate into vehicles molecules of biological interest, for example, polypeptides, proteins, nucleic acids, etc. In a particular embodiment, said whole IBDV VLPs can be used in the development of vaccines to protect birds against the virus causing the avian disease known as infectious bursal disease (IBDV). Virtually any bird, preferably those avian species of economic interest, for example, chickens, turkeys, ganders, geese, pheasants, partridges, ostriches, etc., can be immunized against the infection caused by IBDV with the vaccines provided by this invention. In another particular embodiment, said whole IBDV VLPs can internally incorporate into vehicles products with biological activity, for example, nucleic acids, peptides, proteins, drugs, etc., whereby they can be used in the manufacture of gene therapy vectors.

Therefore, in a further aspect, the present invention is related to the use of said whole IBDV VLPs in the manufacture of medicaments, such as vaccines and gene therapy vectors. Said vaccines and vectors constitute further aspects of the present invention. In a particular embodiment, said vaccine is a vaccine useful for protecting birds from the infection caused by IBDV. In a specific embodiment, said birds are selected from the group formed by chickens, turkeys, ganders, geese, pheasants, partridges, ostriches, preferably chickens.

In another aspect, the invention is related to a process for the production of recombinant baculoviruses useful for the production of whole IBDV VLPs. In a particular embodiment, the recombinant obtained baculoviruses are dual, i.e. the same recombinant baculovirus is able to express in suitable host cells the viral polyprotein and the IBDV VP1 protein from two ORFs, independent and controlled by promoters of different baculoviruses. In another particular embodiment, recombinant baculoviruses are obtained which are able to express in suitable host cells the viral polyprotein from a nucleic acid sequence comprising the ORFs corresponding to the IBDV polyprotein under the control of a promoter, and recombinant baculoviruses able to express in suitable host cells the IBDV VP1 protein from a nucleic acid sequence comprising the ORF corresponding to the IBDV VP1 under the control of a promoter, the same as or different from the one controlling the expression of the viral polyprotein in said recombinant baculoviruses able to express the viral polyprotein. The resulting recombinant baculoviruses constitute a further aspect of the present invention. Said rBVs can be used for the production of whole IBDV VLPs.

### BRIEF DESCRIPTION OF THE FIGURES

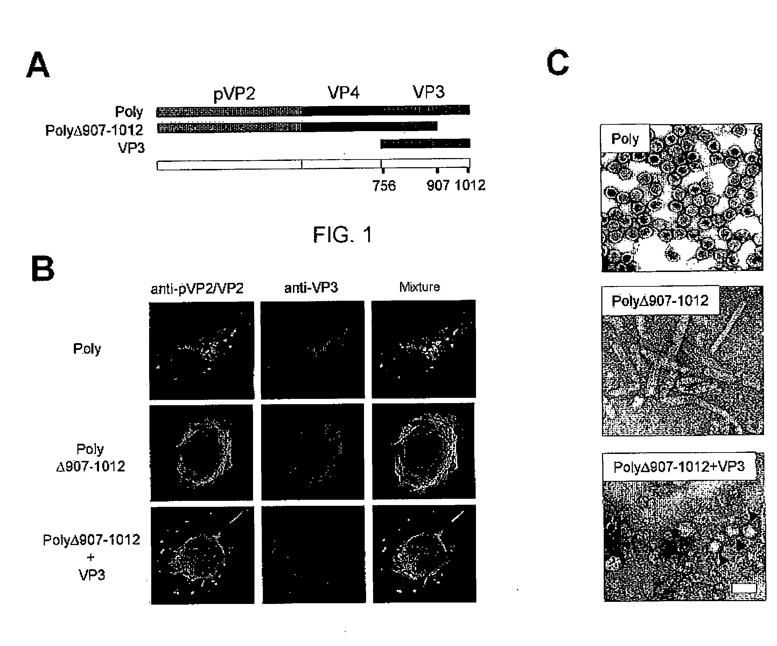

Figure 1 shows the effect of the C-terminal deletion of the IBDV VP3 in the morphogenesis of VLPs. Figure 1A shows a diagram which graphically represents the genes derived from IBDV expressed by the different recombinants of the vaccine virus [VT7/Poly (Poly), disclosed by Fernandez-Arias et al. (Fernandez-Arias A et al. 1998. Expression of ORF A1 of infectious bursal disease virus results in the formation of virus-like particles. J. Gen. Virol. 79:1047-1054), VT7/PolyΔ907-1012 (PolyΔ907-1012) and VT7/VP3 (VP3)] used for checking the effect of the C-terminal end deletion of VP3 in the formation of IBDV VLPs in mammal cells. VT7/Poly (Poly) expresses the whole polyprotein. VT7/PolyΔ907-1012 (PolyΔ907-1012) expresses a deleted form of the polyprotein lacking the 150 C-terminal residues. VT7/VP3 (VP3) expresses the whole VP3 polyprotein. Figure 1B illustrates the effect of the deletion of the C-terminal end of the IBDV polyprotein on the subcellular distribution of the VPX (pVP2) and VP2 proteins, and includes digital confocal microscopy images obtained from infected cells with the recombinants VT7/Poly (Poly), VT7/PolyΔ907-1012 (PolyΔ907-1012) and VT7/VP3 (VP3), respectively. The cells were fixed at 24 hours post-infection (h.p.i.) and incubated with anti- IBDV VPX/2 (anti-pVP2/VP2) rabbit serum and with anti- IBDV VP3 rat serum, followed by incubation with anti-rabbit IgG goat immunoglobulin coupled to Alexa 488 (green) and with anti-rat IgG goat immunoglobulin coupled to Alexa 488 (red). Figure IC shows the effect of the deletion of the C-terminal end of the IBDV polyprotein on the assembly of the capsids; cell extracts infected with VT7/Poly (Poly), VT7/PolyΔ907-1012 (PolyΔ907-1012) or coinfected with VT7/PolyΔ907-1012 (PolyΔ907-1012) and VT7/VP3 (VP3) were subjected to fractioning on sucrose gradient. An aliquot of each one of the fractions was placed on an electron microscopy grid, negatively stained and viewed by means of electron microscopy. The images represent the assemblies detected in equivalent fractions of the different gradients.

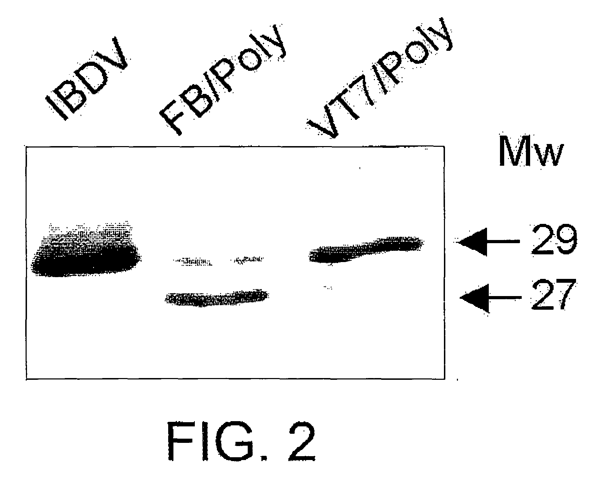

Figure 2 shows the results of a comparative analysis by means of Western blot of the IBDV VP3 protein expressed in different expression systems; cell extracts infected with IBDV, VT7/Poly and FB/Poly, respectively, were subjected to sodium dodecylsulfate polyacrylamide gel electrophoresis (SDS-PAGE) and Western blot analysis using anti-IBDV VP3 rabbit serum, followed by incubation with goat immunoglobulin coupled to peroxidase (HRPO: horse radish peroxidase). The signal was detected by means of ECL (Enhanced Chemiolumineseence). The position of the immunoreactive bands and those of the molecular weight markers arc indicated.

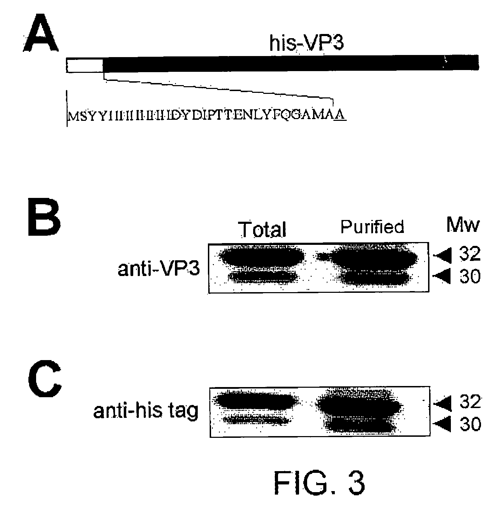

Figure 3 shows the characterization of C-terminal proteolysis of the IBDV VP3 protein expressed in insect cells. Figure 1A shows a diagram graphically representing the his-VP3 gene containing a histidine tag fused to the M-terminal end of VP3 expressed by the recombinant baculovirus FB/his-VP3 [occasionally called in this description FB/his-VP3 wt (wild type)]. The sequence corresponding to the histidine tag and the first amino acid residue corresponding to VP3 (underlined) is indicated. Samples corresponding to whole H5 cell extracts (GIBCO), also identified in this description as H5 cells, infected with FB/his-VP3, or to the his-VP3 protein purified by affinity were subjected to SDS-PAGE and Western blot analysis using anti-VP3 rabbit serum (Figure 1B) or anti-histidine tag (anti-his tag) (Figure 1C) followed by incubation with goat immunoglobulin coupled to peroxidase. The signal was detected by means of ECL. The position of the immunoreactive bands and those of the molecular weight markers are indicated.

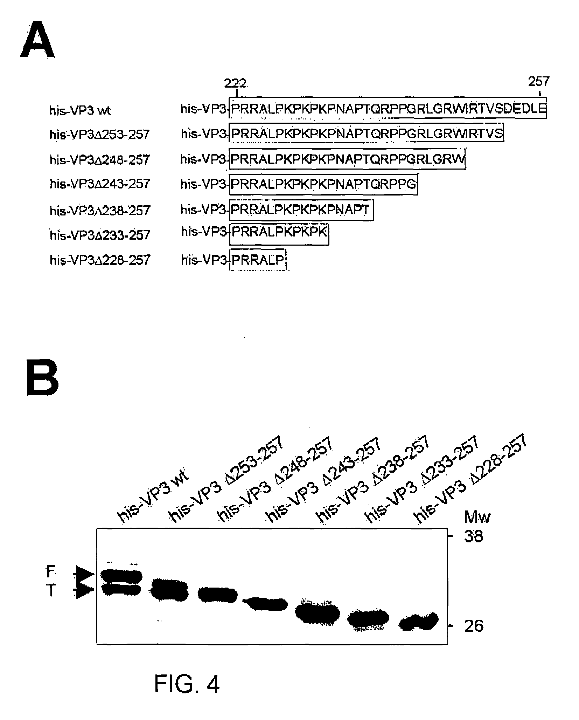

Figure 4 shows the location of the proteolytic cutting site of the IBDV VP3 protein in insect cells. Figure 1A is a diagram graphically representing the group of deleted his-VP3 proteins used in the determination of the position of the proteolytic cutting site of the IBDV VP3 protein in insect cells. Figure 1B shows the result of a Western blot analysis of the different deleted his-VP3 proteins expressed in H5 cells and purified by immobilized metal affinity chromatography (IMAC). H5 cell culture extracts infected with each one of the recombinant baculoviruses were subjected to purification in HiTrap affinity columns (Amersham Pharmacia Biotech). The purified proteins were subjected to SDS-PAGE and Western blot analysis using anti-VP3 rabbit serum, followed by incubation with goat immunoglobulin coupled to peroxidase. The signal was detected by means of ECL. The position of the immunoreactive bands and those of the molecular weight markers are indicated. The arrows indicate the position of the whole protein (F) and the one corresponding to the proteolyzed form (T).

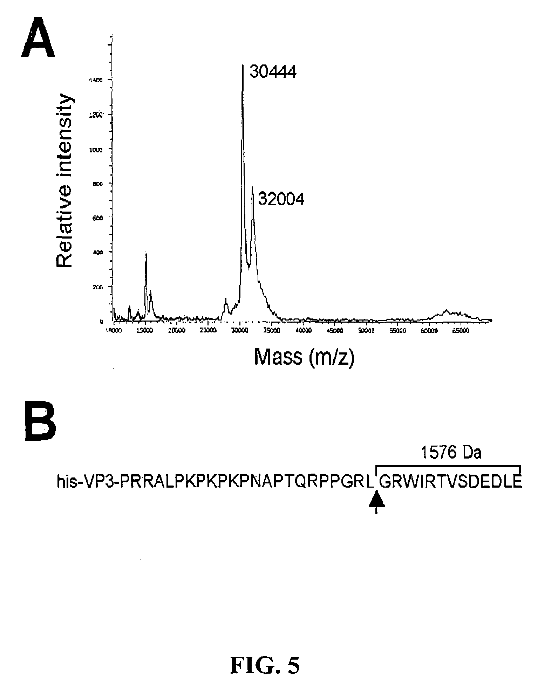

Figure 5 illustrates that the proteolytic processing of IBDV VP3 in insect cells causes the elimination of a peptide of 1.560 Da from the C-terminal end of his-VP3. H5 cell extracts infected with FB/his-VP3 were subjected to purification by means of IMAC and the resulting purified protein was analyzed by means of mass spectrophotometry in triplicate. Figure 5A shows the results of one of these experiments. The presence of two polypeptides of 32.004 and 30.444 Da, respectively, was determined, which proves that the proteolytic processing produces the elimination of a peptide of 1.560 Da from the C-terminal end of his-VP3, size which fits with the molecular mass (1.576 Da) corresponding to the 13 C-terminal residues ofIBDV VP3, the sequence of which is shown in Figure 5B.

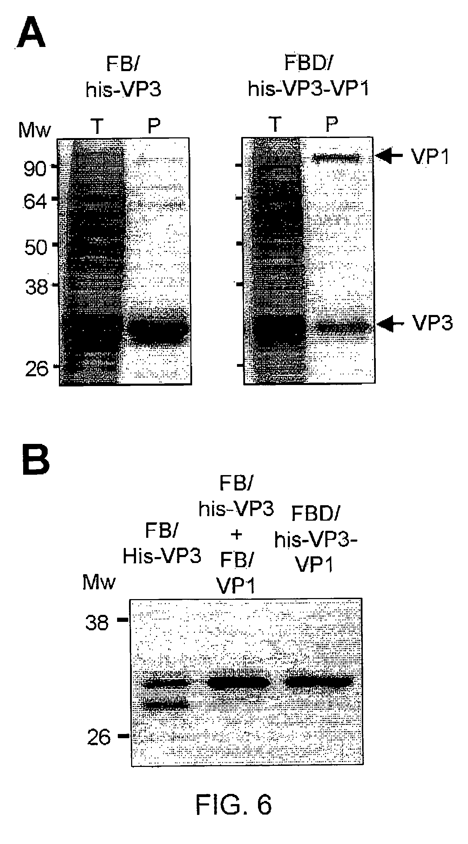

Figure 6 shows the effect of the coexpression of IBDV VP1 on the proteolysis of his-VP3. Figure 6A shows the detection of VP3/VP1 complexes. H5 cells were infected with FB/his-VP3 or with FBD/his-VP3-VP1. At 72 h.p.i., the cells were harvested and the corresponding extracts subjected to purification in HiTrap affinity columns (Amersham Pharmacia Biotech). Samples corresponding to total extracts (T) or to purified proteins were subjected to SDS-PAGE. The gels were subsequently stained with silver nitrate. The position of the molecular weight markers is indicated. Figure 6B shows the results of a Western blot analysis of extracts of H5 cells infected with FB/his-VP3, FBD/his-VP3-VP1, or coinfected with FB/his-VP3 and FB/VP1, respectively. The infected cells were harvested at 72 h.p.i. and homogenized. The corresponding extracts were subjected to SDS-PAGE and Western blot analysis using anti-VP3 rabbit serum, followed by incubation with goat immunoglobulin coupled to peroxidase. The signal was detected by means of ECL. The position of the molecular weight markers is indicated.

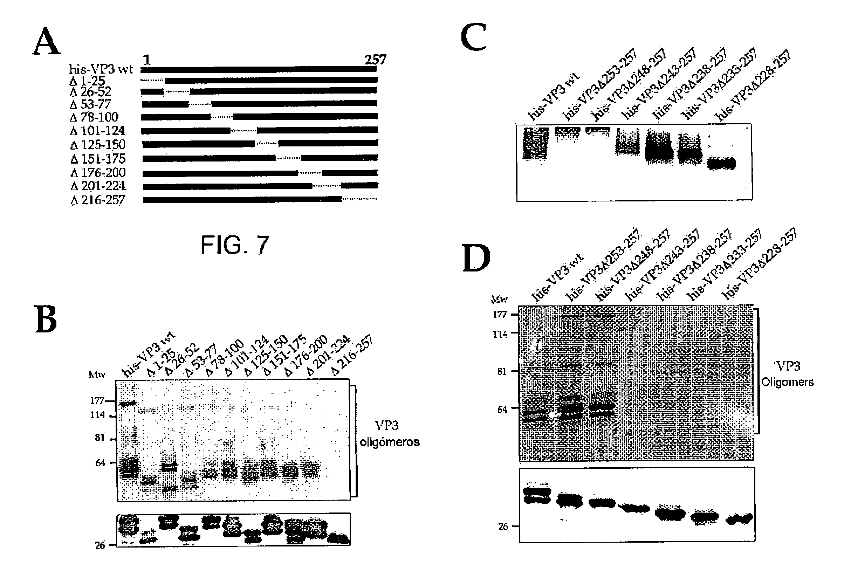

Figure 7 shows the location of the oligomerization domain. Figure 7A is a diagram graphically representing the group of deleted his-VP3 proteins used in the determination of the VP3 oligomerization domain position. The deleted regions are indicated with the dotted line. The name of each mutant indicates the location of eliminated amino acid remains in the sequence of the IBDV VP3 protein. Figure 7B shows the detection of VP3 oligomers. The different his-VP3 deletion proteins, purified by HiTrap affinity columns (Amersham Pharmacia Biotech), were subjected to SDS-PAGE and Western blot analysis using anti-VP3 rabbit serum, followed by incubation with goat immunoglobulin coupled to peroxidase. Figure 1C shows the results of a Western blot analysis. The samples described in the previous paragraph (Figure 7B) were subjected to non-denaturing electrophoresis followed by Western blot analysis using anti-VP3 rabbit scrum, followed by incubation with goat immunoglobulin coupled to peroxidase. Figure 7D shows the detection of VP3 oligomers produced by VP3 C-terminal deletion mutants. The purified proteins were subjected to SDS-PAGE and Western blot analysis using anti-VP3 rabbit serum, followed by incubation with goat immunoglobulin coupled to peroxidase. The signal was detected by means of ECL. The position of the molecular weight markers is indicated.

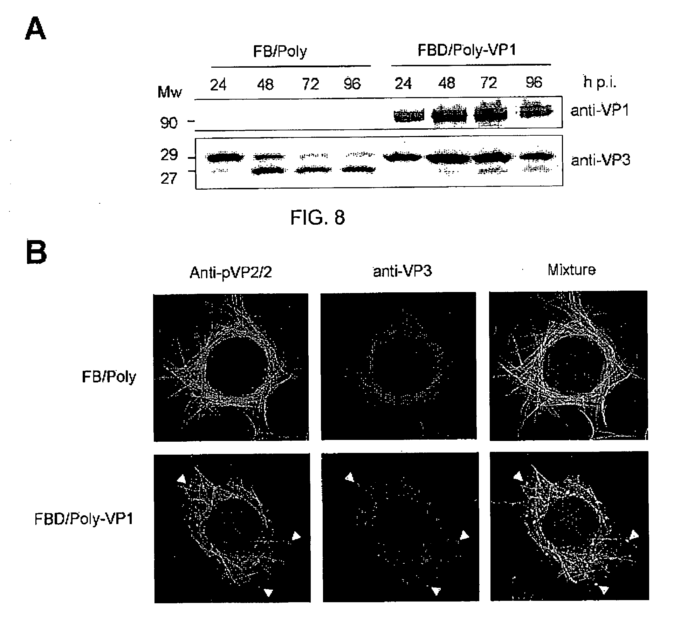

Figure 8 shows the determination of the effect of the coexpression of IBDV VP1 on the proteolytic processing of IBDV VP3 and the subcellular distribution of the proteins of the capsid. Figure 8A illustrates the detection of the IBDV VP1 and VP3 proteins accumulated in H5 cells infected with FB/Poly and FBD/Poly-VP1, respectively. Infected cells were harvested at 24, 48 and 72 h.p.i. The samples were subjected to SDS-PAGE and Western blot analysis using anti-VP3 or anti-VP1 rabbit serum, followed by incubation with goat immunoglobulin coupled to peroxidase. The position of the molecular weight markers is indicated. The subcellular distribution of the VPX/2 (pVP2/VP2) and VP3 proteins in cells infected with FB/Poly and FBD/Poly-VP1 was analyzed by confocal microscopy (Figure 8B). The cells were fixed at 60 h.p.i., and then incubated with anti-VPX rabbit serum (anti-pVP2) and anti-VP3 rat scrum followed by incubation with anti-rabbit IgG goat immunoglobulin coupled to Alexa 488 (green) and with anti-rat IgG goat immunoglobulin coupled to Alexa 488 (red). The arrows indicate the position of the viroplasms formed by VPX/2 (pVP2/VP2) and VP3.

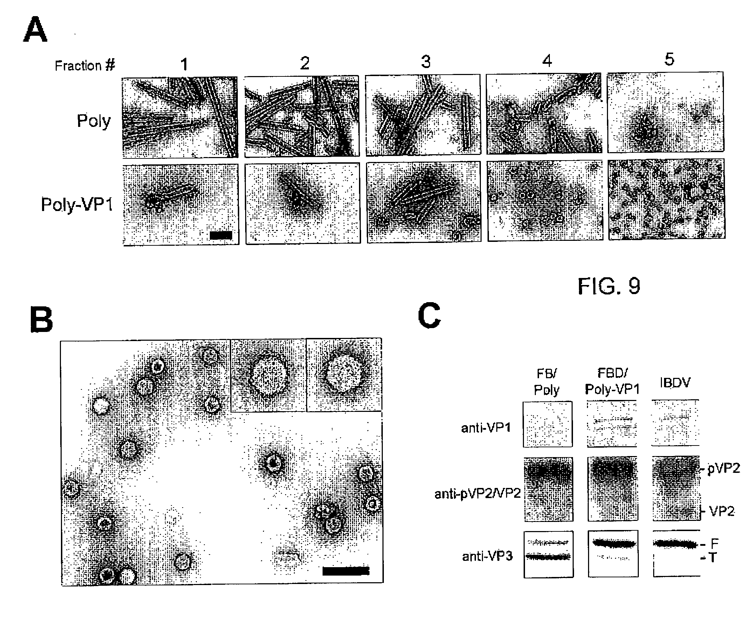

Figure 9 illustrates the characterization of the structures formed by expression of the IBDV polyprotein in cells infected with FB/Poly-VP1. Figure 9A shows a set of micrographs of the structures obtained in the different fractions. H5 cells were infected with FB/Poly (Poly) or with FBD/Poly-VP1 (Poly-VP1). The cells were harvested at 90 h.p.i. and the corresponding extracts were used for the purification of structures by means of sucrose gradients. After centrifugation, 6 aliquots of 2 ml were taken. One part of the aliquot was placed on a grid, negatively stained with uranyl acetate, and analyzed by means of observation in the electron microscope. Fractions #1 correspond to the bottom of the gradients. Fractions #6, which contained soluble protein and de-assembled structures, are not shown. Die bar corresponding to 200 nm. Figure 9B is a micrograph showing purified VLPs from cells infected with FBD/Poly-VP1. The image corresponds to fraction #5 of the gradient obtained from cells infected with FBD/Poly-VP1. The enlarged boxes show 2 VLPs at a larger amplification. Figure 9C shows the characterization of the polypeptides present in fraction #5 of both gradients. An aliquot of fraction #5 of each gradient was subjected to SDS-PAGE and Western blot analysis using anti-VP1, anti-VPX (anti-pVP2/VP2) or anti-VP3 rabbit scrum, followed by incubation with goat immunoglobulin coupled to peroxidase. The position of VPX (pVP2), VP2, whole VP3 (F) and proteolyzed VP3 (T) is shown.

### DETAILED DESCRIPTION OF THE INVENTION

In a first aspect, the invention provides a whole empty viral capsid of the infectious bursal disease virus (IBDV), hereinafter whole IBDV VLP (whole VLPs in plural form) of the invention, characterized in that it contains all the proteins present in purified and infective IBDV virions, specifically the IBDV VPX, VP2, VP3 and VP1 proteins.

The term "IBDV", as it is used in the present invention, refers to the different IBDV strains belonging to any of the serotypes (1 or 2) known [by way of illustration, see the review carried out by van den Berg TP, Eterradossi N, Toquin D, Meulemans G., in *Rev Sci Tech* 2000 19:509-43].

The terms "viral polyprotein" or "IBDV polyprotein" are indistinctly used in this description and refer to the product resulting from the expression of the A segment of the IBDV genome the proteolytic processing of which gives rise to the VPX (pVP2), VP3 and VP4 proteins, and include the different forms of the polyproteins representative of any of the mentioned IBDV strains [NCBI protein databank], according to the definition carried out by Sánchez and Rodríguez (1999) (Sánchez AB & Rodríguez JF. Proteolytic processing in infectious bursal disease virus: identification of the polyprotein cleavage sites by site-directed mutagenesis. Virology. 1999 Sep 15; 262(1):190-199), as well as proteins substantially homologous to said IBDV polyprotein, i.e. proteins the amino acid sequences of which have a degree of identity regarding said IBDV polyprotein of at least 60%, preferably of at least 80%, more preferably of at least 90% and even more preferably of at least 95%.

The term "IBDV VP1 protein" refers to the product resulting from the expression of segment B of the IBDV genome and includes the different forms of the VP1 proteins representative of any of the mentioned IBDV strains [NCBI protein databank], according to the definition carried out by Lombardo E ct al. 1999. VP1, The putative RNA-dependent RNA polymerase of infectious bursal disease virus, forms complexes with the capsid protein VP3, leading to efficient encapsidation into virus-like particles. J. Virol. 73:6973-83) as well as proteins substantially homologous to said IBDV VP1 protein, i.e. proteins the amino acid sequences of which have a degree of identity regarding said IBDV VP1 of at least 60%, preferably of at least 80%, more preferably of at least 90% and even more preferably of at least 95%.

The IBDV VPX (pVP2), VP2 and VP3 proteins present in the whole IBDV VLPs of the invention can be any of the VPX, VP2 and VP3 proteins representative of any IBDV strain obtained by proteolytic processing of the viral polyprotein, for example the IBDV Soroa strain VPX, VP2 and VP3 proteins [NCBI, access number AAD30136].

The IBDV VP1 protein present in the whole IBDV VLPs of the invention can be any VP1 protein representative of any IBDV strain, for example, the whole length, Soroa strain VP1 protein, the amino acid sequence of which is shown in SEQ. ID. NO: 2.

In a particular embodiment, the whole IBDV VLPs of the invention have a diameter of 65-70 nm and a polygonal contour indistinguishable from the IBDV virions.

The whole IBDV VLPs of the invention can be obtained by means of the simultaneous expression of said IBDV viral polyprotein and VP1 protein in suitable host cells. Said suitable host cells are cells containing the encoding nucleotide sequence of the IBDV polyprotein under the control of a suitable promoter and the encoding nucleotide sequence of the IBDV VP1 protein under the control of another suitable promoter, either in a single gene construct or in two different gene constructs. In a particular embodiment, said suitable host cells are cells that are transformed, transfected or infected with a suitable expression system, such as (1) an expression system comprising a gene construct, in which said gene construct comprises the nucleotide sequence encoding for the IBDV polyprotein under the control of a promoter and the encoding nucleotide sequence of the IBDV VP1 protein under the control of another promoter different from the one which is operatively bound to the nucleotide sequence encoding the viral polyprotein, or, alternatively, (2) an expression system comprising a first gene construct comprising the nucleotide sequence encoding for the IBDV polyprotein, and a second gene construct comprising the nucleotide sequence encoding for the IBDV VP1 protein, each one of them under the control of a suitable promoter. In a particular embodiment, said host cell is an insect cell and said promoters are baculovirus promoters.

Therefore, in another aspect, the invention is related to a gene construct comprising the nucleotide sequence encoding for said IBDV polyprotein and the nucleotide sequence encoding for said IBDV VP1 protein, in the form of two independent ORFs, the expression of which is controlled by respective different promoters controlling the gene expression of each one of said IBDV viral polyprotein and VP1 protein. Therefore, the invention provides a gene construct comprising (i) a nucleotide sequence comprising the open reading frames corresponding to the polyprotein of the infectious bursal disease virus (IBDV) operatively bound to a nucleotide sequence comprising a first promoter and (ii) a nucleotide sequence comprising the open reading frame corresponding to the TBDV VP1 protein operatively bound to a nucleotide sequence comprising a second promoter, in which said first promoter is different from said second promoter. The use of said different promoters allows the independent and simultaneous control of the gene expression of said IBDV polyprotein and VP1 protein.

A feature of the gene construct provided by this invention is that it comprises the nucleotide sequences encoding for all the protein elements present in the purified and infective IBDV virions, specifically, the VPX, VP2, VP3 and VP1 proteins.

As it is used in this description, the term "ORFs (or open reading frames) corresponding to the IBDV polyprotein" or "ORF (open reading frame) corresponding to the IBDV VP1 protein" includes, in addition to the nucleotide sequences of said ORFs, other ORFs analogous to the same encoding sequences of the IBDV viral polyprotein and of the IBDV VP1. The term "analogous", as it is used herein, intends to include any nucleotide sequence which can be isolated or constructed on the base of the encoding nucleotide sequence of the viral polyprotein and the IBDV VP1, for example, by means of the introduction of conservative or non-conservative nucleotide replacements, including the insertion of one or more nucleotides, the addition of one or more nucleotides at any end of the molecule, or the deletion of one or more nucleotides at any end or inside of the sequence. Generally, a nucleotide sequence analogous to another nucleotide sequence is substantially homologous to said nucleotide sequence. In the sense used in this description, the expression "substantially homologous" means that the nucleotide sequences in question have a degree of identity, at the nucleotide level, of at least 60%, advantageously of at least 70%, preferably of at least 80%, more preferably of at least 85%, even more preferably of at least 90%, and yet even more preferably of at least 95%.

The promoters which can be used in the implementation of the present invention generally comprise a nucleic acid sequence to which the RNA polymerase is bound so as to begin the mRNA transcription and to express said ORFs corresponding to the viral polyprotein and to the TBDV VP1 protein in suitable host cells. Although virtually any promoter meeting these conditions can be used to implement the present invention, for example, promoters of a viral, bacterial, yeast, animal, plant origin, etc., in a particular embodiment said promoters are viral promoters, for example, baculovirus promoters.

The expression of each one of said nucleotide sequence encoding for said viral polyprotein and IBDV VP1 protein, in the form of two independent ORFs, is controlled by respective different promoters controlling the gene expression of each one of said proteins. In a particular embodiment, the gene expression of said viral polyprotein and IBDV VP1 protein is carried out in insect cells infected or coinfected with recombinant baculoviruses (rBVs) containing the encoding nucleotide sequences of said proteins, either in a single rBV (dual rBV) or in two rBVs (in which case one of said rBVs contains the encoding sequence of the IBDV polyprotein and the other one, the encoding sequence of the IBDV VP1 protein) under the control of baculovirus promoters.

Virtually any baculovirus promoter can be used as long as it is able to effectively control the expression of the encoding sequence to which it is operatively bound. By way of illustration, said first baculovirus promoter can be the promoter of the p10 protein of the baculovirus *Autographa californica* nucleopolyhedrovirus (AcMNV), the promoter of the polyhedrin of the AcMNPV baculovirus, etc. and said second baculovirus promoter can be the promoter of the p10 protein of AcMNPV and the promoter of the AcMNPV polyhedrin. More specifically, in a particular embodiment, said first baculovirus promoter is the promoter of the p10 protein of AcMNPV and said second baculovirus promoter is the promoter of the AcMNPV polyhedrin, whereas in another particular embodiment, said first baculovirus promoter is the promoter of the AcMNPV polyhedrin and said second baculovirus promoter is the promoter of the protein 10 of AcMNPV.

In a particular embodiment, the gene construct provided by this invention comprises:
(i) a nucleotide sequence comprising the open reading frames corresponding to the IBDV polyprotein operatively bound to a nucleotide sequence comprising a first promoter of a baculovirus, and
(ii) a nucleotide sequence comprising the ORF corresponding to the IBDV VP1 protein operatively bound to a nucleotide sequence comprising a second promoter of a baculovirus,

wherein said first and second baculovirus promoters are different.

The use of different baculovirus promoters allows for the independent and simultaneous control of the gene expression of said IBDV polyprotein VP1 protein in insect cells.

In a specific embodiment, the gene construct provided by this invention comprises the encoding sequence of the IBDV polyprotein under the control of a first baculovirus promoter and the encoding sequence of the IBDV VP1 protein under the control of a second baculovirus promoter, different from the first one, such as the gene construct referred to as "Poly-VP1" in this description, comprising the nucleotide sequence identified as SEQ. ID. NO: 1; said Poly-VP1 gene construct contains the encoding sequence of the IBDV polyprotein under the control of the promoter of the AcMNV polyhedrin and the encoding sequence of the IBDV VP1 protein under the control of the promoter of the AcMNV p10 protein.

In another aspect, the invention provides an expression vector or system selected from:
a) an expression system comprising a gene construct provided by this invention, operatively bound to transcription, and optionally translation, control elements, wherein said gene construct comprises (i) a nucleotide sequence comprising the ORFs corresponding to the IBDV polyprotein operatively bound to a nucleotide sequence comprising a first promoter and (ii) a nucleotide sequence comprising the ORF corresponding to the IBDV VP1 protein operatively bound to a nucleotide sequence comprising a second promoter, wherein said first promoter is different from said second promoter; and
b) an expression system comprising (1) a first gene construct, operatively bound to transcription, aad optionally translation, control elements, wherein said first gene construct comprises a nucleotide sequence comprising the ORFs corresponding to the IBDV polyprotein operatively bound to a nucleotide sequence comprising a first promoter, and (2) a second gene construct, operatively bound to transcription, and optionally translation, control elements, wherein said second gene construct comprises a nucleotide sequence comprising the ORF corresponding to the IBDV VP1 protein operatively bound to a nucleotide sequence comprising a second promoter.

In the second case [b)], said first promoter and said second promoter, as they are in different gene constructs, can be equal to or different from one another.

The features of the ORFs corresponding to the IBDV polyprotein and to the IBDV VP1 protein have previously been defined in relation to the gene construct provided by this invention. The promoters which can be used in the expression system provided by this invention have been previously defined in relation to the gene construct provided by this invention. By way of illustration, said promoters can be promoters of a viral, bacterial, yeast, animal, plant origin, etc.

In a particular embodiment, the expression system provided by this invention comprises a gene construct, operatively bound to transcription, and optionally translation, control elements, wherein said gene construct comprises (i) a nucleotide sequence comprising the ORFs corresponding to the IBDV polyprotein operatively bound to a nucleotide sequence comprising a first baculovirus promoter, such as, for example, the promoter of the AcMN V p10 protein or the promoter of the AcMNV polyhedrin, and (ii) a nucleotide sequence comprising the ORF corresponding to the IBDV VP1 protein operatively bound to a nucleotide sequence comprising a second baculovirus promoter, such as, for example, the promoter of the AcMNV p10 protein or the promoter of the AcMNV polyhedrin, wherein said first baculovirus promoter is different from said second baculovirus promoter.

In another particular embodiment, the expression system provided by this invention comprises (1) a first gene construct, operatively bound to transcription, and optionally translation, control elements, wherein said first gene construct comprises a nucleotide sequence comprising the ORFs corresponding to the IBDV polyprotein operatively bound to a nucleotide sequence comprising a first baculovirus promoter, such as, for example, the promoter of the AcMNV p10 protein or the promoter of the AcMNV polyhedrin, and (2) a second gene construct, operatively bound to transcription, and optionally translation, control elements, wherein said second gene construct comprises a nucleotide sequence comprising the ORF corresponding to the IBDV VP1 protein operatively bound to a nucleotide sequence comprising a second baculovirus promoter, such as, for example, the promoter of the AcMNV p10 protein or the promoter of the AcMNV polyhedrin. In this particular embodiment, said first baculovirus promoter and said second baculovirus promoter, as they are in different gene constructs, can be equal to or different from one another.

The transcription, and optionally translation, control elements present in the expression system provided by this invention include the necessary or suitable sequences for the transcription and its suitable control in time and place, for example, beginning and termination signals, cleavage sites, polyadenylation signals, replication origin, transcriptional activators (enhancers), transcriptional silencers (silencers), etc.

Virtually any suitable expression system or vector can be used in the generation of the expression system provided by this invention depending on the conditions and requirements of each specific case. By way of illustration, said suitable expression systems or vectors can be plasmids, bacmids, yeast artificial chromosomes (YACs), bacteria artificial chromosomes (BACs), bacteriophage P1-based artificial chromosomes (PACs), cosmids, viruses, which can further have, if so desired, an origin of heterologous replications, for example, bacterial, so that it may be amplified in bacteria or yeasts, as well as a marker usable for selecting the transfected cells, etc., preferably plasmids, bacmids or viruses.

These expression systems or vectors can be obtained by conventional methods known by persons skilled in the art [Sambrook, J., Fritsch, E.F., and Maniatis, T. (1989). Molecular cloning: a laboratory manual, 2nd ed. Cold Spring Harbor Laboratory] and form part of the present invention. In a particular embodiment, said expression system or vector is a plasmid, such as the plasmid referred to as pFBD/Poly-VP1 in this description, or a bacmid, such as the recombinant bacmid referred to as Bac/pFBD/Poly-VP1 in this description, which contain the previously defined gene construct Poly-VP1, or a virus, such as the recombinant baculovirus (rBV) referred to as FBD/Poly-VP1 in this description, which contains the gene construct Poly-VP1 and expresses during its replication cycle both proteins (polyprotein and IBDV VP1 protein) simultaneously in insect cells, or the rBVs expressing the IBDV polyprotein and the IBDV VP1 protein, seperately and simultaneously, when coinfecting insect cells, whole IBDV VLPs being obtained.

In another aspect, the invention provides a host cell containing the encoding nucleotide sequence of the IBDV polyprotein and the encoding nucleotide sequence of the IBDV VP1 protein, each one of them under the control of a suitable promoter allowing the simultaneous and independent control of said IBDV polyprotein and VP1 protein, either in a single gene construct (in which case the promoters bound to each one of said encoding sequences would be different from one another), or in two different gene constructs. Therefore, said host cell can contain either a gene construct provided by this invention or an expression system provided by this invention.

The host cell provided by this invention can be a host cell transformed, transfected or infected with an expression system provided by this invention.

In a particular embodiment, the host cell provided by this invention is a host cell transformed, transfected or infected with an expression system provided by this invention comprising a gene construct, operatively bound to transcription, and optionally translation, control elements, wherein said gene construct comprises (i) a nucleotide sequence comprising the ORFs corresponding to said IBDV polyprotein operatively bound to a nucleotide sequence comprising a first promoter and (ii) a nucleotide sequence comprising the open reading frame corresponding to said IBDV VP1 protein operatively bound to a nucleotide sequence comprising a second promoter, wherein said first promoter is different from said second promoter.

Alternatively, in another particular embodiment, said host cell is a host cell transformed, transfected or infected with an expression system provided by this invention comprising (1) a first gene construct, operatively bound to transcription, and optionally translation, control elements, wherein said first gene construct comprises a nucleotide sequence comprising the ORFs corresponding to said IBDV polyprotein operatively bound to a nucleotide sequence comprising a first promoter, and (2) a second gene construct, operatively bound to transcription, and optionally translation, control elements, wherein said second gene construct comprises a nucleotide sequence comprising the ORF corresponding to the IBDV VP1 protein operatively bound to a nucleotide sequence comprising a second promoter; in this particular embodiment, said first promoter and said second promoter, as they are in different gene constructs, can be equal to or different from one another.

Although in any of the previously mentioned embodiments, virtually any promoter could be used, it is preferred in practice that said promoters are useful in bacteria, yeasts, viruses, animal cells, for example, in mammal cells, bird cells, insect cells, etc-; in a particular embodiment, said promoters are baculovirus promoters, such as, for example, the promoter of the AcMNV polyhedrin or the promoter of the AcMNV p10 protein.

Virtually any host cell susceptible to being transformed, transfected or infected by an expression system provided by this invention can be used, for example, bacteria, mammal cells, bird cells, insect cells, etc.

In a particular embodiment, said host cell is a bacteria transformed with an expression system provided by this invention comprising a gene construct provided by this invention comprising (i) a nucleotide sequence comprising the ORFs corresponding to the IBDV polyprotein and (ii) a nucleotide sequence comprising the CRTs Corresponding to the IBDV VP1 protein, each one of them operatively bound to a different promoter, such as the gene construct identified as Poly-VP1. A culture of *Escherichia coli* bacteria strain DH5, transformed with said gene construct Poly-VP1, and identified as DH5-pFBD/Poly-VP1 has been deposited in the Spanish Type Culture Collection (hereinafter, CFCT) with deposit number CECT 5777.

Alternatively, said host cell is an insect cell. Insect cells are suitable when the expression system comprises one or more rBVs. The use of rBVs is advantageous due to biosafety issues related to the host range of the baculoviruses, incapable of replicating in other cell types which are not insect cells.

Therefore, in a particular embodiment, the invention provides a host cell, such as an insect cell, infected with an expression system provided by this invention, such as a rBV, comprising a gene construct provided by this invention comprising (i) a nucleotide sequence comprising the ORFs corresponding to the IBDV polyprotein and (ii) a nucleotide sequence comprising the ORF corresponding to the IBDV VP1 protein, each one of them operatively bound to a different baculovirus promoter, such as the gene construct identified as Poly-VP1.

In another particular embodiment, the invention provides host cell, such as an insect cell, coinfected with an expression system comprising (1) a first rBV comprising a gene construct comprising the ORFs corresponding to said IBDV polyprotein and (2) a second rBV comprising a gene construct comprising the nucleotide sequence comprising the ORF corresponding to said IBDV VP1 protein, each one of said encoding sequences being operatively bound to a baculovirus promoter, equal to or different from one another.

In another aspect, the invention provides a process for producing whole IBDV VLPs of the invention comprising culturing a host cell provided by this invention containing a nucleotide sequence comprising the ORFs corresponding to said IBDV polyprotein and a nucleotide sequence comprising the ORF corresponding to said IBDV VP1 protein, either in a single gene construct or in two different gene constructs, and simultaneously expressing said viral polyprotein and IBDV VP1 protein, and if so desired, recovering said whole IBDV VLPs of the invention.

In a particular embodiment, said host cell provided by this invention is a cell transformed, transfected or infected with a suitable expression system provided by this invention, such as an expression system comprising a gene construct provided by this invention, wherein said gene construct comprises (i) a nucleotide sequence comprising the ORFs corresponding to said IBDV polyprotein operatively bound to a nucleotide sequence comprising a first promoter and (ii) a nucleotide sequence comprising the ORF corresponding to said IBDV VP1 protein operatively bound to a nucleotide sequence comprising a second promoter, wherein either said first promoter is different from said second promoter; or alternatively, with an expression system provided by this invention comprising (1) a first gene construct comprising a nucleotide sequence comprising the ORFs corresponding to said IBDV polyprotein and (2) a second gene construct comprising a nucleotide sequence comprising the ORF corresponding to said IBDV VP1 protein, each one of said nucleotide sequences comprising the ORFS corresponding to the viral palyprotein and to the IBDV VP1 protein being under the control of respective nucleotide sequences comprising respective promoters, equal to or different from one another.

Said process therefore comprises the simultaneous gene coexpression of said viral polyprotein and IBDV VP1 protein as two independent ORFs. After the simultaneous expression of said viral polyprotein and VP1 protein in said cells, the polyprotein is proteolytically processed and the resulting proteins arc assembled and form the whole IBDV VLPs of the invention, made up of VPX, VP2, VP3 and VP1, which can be isolated or withdrawn from the medium and, if desired, purified. The isolation or purification of said whole IBDV VLPs of the invention can be carried out by means of conventional methods, for example, by means of fractioning on sucrose gradients.

Although the host cell to culture can be any of those previously defined, in a particular embodiment, said host cell is an insect cell.

Therefore, in a specific embodiment, the simultaneous gene coexprcssion of the viral polyprotein and of the IBDV VP1 protein in a suitable host cell, such as an insect cell, is carried out by means of the use of a dual rBV allowing the simultaneous expression of said proteins from two independent ORFs, each one of them under the control of a different baculovirus promoter able to simultaneously and independently control the expression of said proteins in insect cells. In this case, the production of the whole IBDV VLPs of the invention can be carried out by means of a process comprising, first, the obtaining of a gene expression system made up of a dual rBV containing a gene construct simultaneously comprising the ORFS corresponding to said viral polyprotein and IBDV VP1 protein, such as the rBV referred to as FBD/Poly-VP1 in this description, or else, alternatively, the obtaining of a rBV containing a gene construct comprising the ORF corresponding to the IBDV polyprotein and the obtaining of another rBV containing a gene construct comprising the ORF corresponding to the IBDV VP1 protein, followed by the infection of insect cells with said expression system based on said rVB(s), expression of the recombinant proteins and, if so desired, isolation of the formed whole IBDV VLPs of the invention, and optionally, subsequent purification of said whole IBDV VLPs of the invention.

More specifically, in a particular embodiment, the process for obtaining whole VLPs of the invention is characterized in that the host cell is an insect cell and comprises the steps of:
a) preparing an expression system provided by this invention made up of (1) a first recombinant baculovirus comprising a gene construct comprising a nucleotide sequence comprising the ORFs corresponding to the IBDV polyprotein operatively bound to a baculovirus promoter, said gene construct being operatively bound to transcription, and optionally translation, control elements, and of (2) a second recombinant baculovirus comprising a gene construct comprising a nucleotide sequence comprising the ORF, corresponding to the IBDV VP1 protein operatively bound to a promoter of a baculovirus, said gene construct being operatively bound to several transcription, and optionally translation, control elements;
b) infecting insect cells with said expression system prepared in step a);
c) culturing the infected insect cells obtained in step b) under conditions allowing the expression of the recombinant proteins and their assembly so as to form whole IBDV VLPs; and
d) if so desired, isolating and optionally purifying said whole IBDV VLPs.

Likewise, in another particular embodiment, the process for obtaining whole VLPs of the invention is characterized in that the host cell is an insect cell and comprises the steps of:
a) preparing an expression system made up of a dual recombinant baculovirus comprising a gene construct comprising (i) a nucleotide sequence comprising the ORFs corresponding to the IBDV polyprotein operatively bound to a nucleotide sequence comprising a first baculovirus promoter, said gene construct being operatively bound to transcription, and optionally translation, control elements, and (ii) a nucleotide sequence comprising the ORF corresponding to the IBDV VP1 protein operatively bound to a nucleotide sequence comprising a second baculovirus promoter, said gene construct being operatively bound to transcription, and optionally translation, control elements, wherein said baculovirus promoter is different from said second baculovirus promoter;
b) infecting insect cells with said expression system prepared in step a);
c) culturing the infected insect cells obtained in step b) under conditions allowing the expression of the recombinant proteins and their assembly so as to form whole IBDV VLPs; and
d) if so desired, isolating and optionally purifying said whole IBDV VLPs.

The construct of a dual rBV simultaneously allowing expression of the IBDV polyprotein and of the IBDV VP3 protein can be carried out by a person skilled in the art based on that herein described and on the state of the art on this technology (Cold Spring Harbor, N.Y.; Leusch MS, Lee SC, Olins PO. 1995. A novel host-vector system for direct selection of recombinant baculoviruses (bacmids) in *Escherichia coli.* Gene 160:191-4; Luckow VA, Lee SC, Barry GF, Olins PO. 1993. Efficient generation of infectious recombinant baculoviruses by site-specific transposon-mediated insertion of foreign genes into a baculovirus genome propagated in *Escherichia coli.* J Virol 67:4566-79). A rBV containing the gene construct comprising the ORFs corresponding to the IBDV polyprotein and a rBV containing a gene construct comprising the ORF corresponding to the IBDV VP1 protein can be similarly obtained.

In relation with this, the invention provides a process for obtaining a dual rBV allowing the simultaneous expression of the IBDV polyprotein and of IBDV VP1 protein from two independent ORFs and each one of them controlled by a different baculovirus promoter, in insect cells, comprising:
a) constructing a plasmid carrier of a gene construct containing (i) a nucleotide sequence comprising the open reading frames corresponding to the IBDV polyprotein operatively bound to a nucleotide sequence comprising a first promoter of a baculovirus, and (ii) a nucleotide sequence comprising the open reading frame corresponding to the IBDV VP1 protein operatively bound to a nucleotide sequence comprising a second promoter of a baculovirus, wherein said first baculovirus promoter is different from said second baculovirus promoter and they allow the simultaneous control of the gene expression of said polyprotein and IBDV VP1 protein;
b) obtaining a recombinant bacmid, simultaneously allowing the expression during its replicative cycle of the polyprotein and the IBDV VP1 protein under the transcriptional control of said baculovirus promoters, by means of the transformation of competent bacteria with the plasmid obtained in a); and
c) obtaining a dual recombinant baculovirus, allowing the simultaneous expression of the open reading frames corresponding to the polyprotein and the IBDV VP1 protein under the transcriptional control of said baculovirus promoters, by means of the transformation of insect cells with the recombinant bacmid of b).

As it is used in this description, the term "competent bacteria" refers to bacteria which can contain the genome of a baculovirus, for example, AcMNV, optionally genetically modified, allowing the recombination with donor plasmids.

In a particular embodiment, said process of obtaining dual rBVs is characterized in that:
- said first baculovirus promoter sequence comprises the promoter of the AcMNV p10 protein and said second baculovirus promoter sequence comprises the promoter of the AcMNPV polyhedrin, or vice versa;
- the plasmid obtained in a) is the one identified as pFBD/Poly-VP1 in this description;
- the competent bacteria transformed in b) are *Escherichia coli* DH10Bac;
- the recombinant bacmid obtained in b) is the one identified as Bac/pFBD/Poly-VP1 in this description; and
- the dual rBV obtained is the one identified as FBD/Poly-VP1.

The dual rBV thus obtained can be used, if so desired, to obtain whole IBDV VLPs of the invention. To that end, insect cells are infected with said dual rBV. Virtually any insect cell can be used; however, in a particular embodiment, said insect cells are H5 cells or *Spodoptera frugiperda* Sf9 cells.

Alternatively, as previously mentioned, whole VLPs of the invention can be obtained by means of the combined infection (coinfection) of insect cells with a rBV allowing expression of the IBDV polyprotein in insect cells and with a rBV allowing expression of the IBDV VP1 protein in insect cells. Said rBVs can be obtained according to that previously mentioned. Virtually any insect cell can be used; however, in a particular embodiment, said insect cells are H5 cells or *Spodoptera frugiperda* Sf9 cells.

Therefore, in another aspect, the invention is related to a process for the production of rBVs useful for the production of whole IBDV VLPs. In a particular embodiment, the recombinant baculoviruses obtained arc dual, i.e., the same recombinant baculovirus is able to express in suitable host cells the viral polyprotein and the IBDV VP1 protein from two independent ORFs and controlled by different baculovirus promoters. The simultaneous expression in the same host cell of said viral polyprotein and IBDV VP1 protein allows the formation of whole IBDV VLPs. In another particular embodiment, recombinant baculoviruses are obtained which are able to express in suitable host cells the viral polyprotein from a nucleic acid sequence comprising the ORFs corresponding to the IBDV polyprotein under the control of a baculovirus promoter and several recombinant baculoviruses able to express in suitable host cells the IBDV VP1 protein from a nucleic acid sequence comprising the ORF corresponding to the VP1 of IBDV under the control of a promoter that is equal to or different from the one regulating the expression of the viral polyprotein in said recombinant baculoviruses able to express the viral polyprotein. The combined infection (coinfection) of suitable host cells, such as insect cells, with said recombinant baculoviruses able to express the viral polyprotein and with said recombinant baculoviruses able to express the IBDV VP1 protein, allows for the simultaneous expression in said coinfected cells of the viral polyprotein and of the IBDV VP1 protein, which allows for the formation of whole IBDV VLPs. The resulting recombinant baculoviruses constitute a further aspect of the present invention.

In another aspect, the invention is related to the use of the gene expression system provided by this invention for the production of whole IBDV VLPs of the invention, which constitute a further aspect of this invention.

The whole IBDV VLPs of the invention can be used to immunize animals, particularly birds, *per se* or as vectors or vehicles of molecules with biological activity, for example, polypeptides, proteins, nucleic acids, drugs, etc., whereby they can be used with therapeutic or diagnostic purposes. In a particular embodiment, said molecules with biological activity include antigens or immune response inducers in animals or humans to whom they are supplied, or drugs which can be released in their specific action site, or nucleic acid sequences, all being useful in gene therapy and intended for being introduced inside the suitable cells.

Therefore, in another aspect, the invention is related to the use of the whole IBDV VLPs of the invention in the manufacture of medicaments such as vaccines, gene therapy vectors (delivery systems), etc. In a particular embodiment, said medicament is a vaccine intended for conferring protection to animals, particularly birds, against the infectious bursal disease virus (IBDV). In another particular embodiment, said medicament is a gene therapy vector.

In another aspect, the invention provides a vaccine comprising a therapeutically effective amount of whole IBDV VLPs of the invention, optionally together with one or more pharmaceutically acceptable adjuvants and/or vehicles. Said vaccine is useful for protecting animals, particularly birds, against the infectious bursal disease virus (IBDV. In a particular embodiment, said birds are selected from the group formed by chickens, turkeys, geese, ganders, pheasants, partridges and ostriches. In a preferred embodiment, the vaccine provided by this invention is a vaccine useful for protecting chickens from the infection caused by IBDV.

In the sense used in this description, the expression "therapeutically effective amount" refers to the amount of whole IBDV VLPs of the invention calculated for producing the desired effect and will generally be determined, among others, by the characteristics of the whole IBDV VLPs of the invention and the immunization effect to be achieved.

The pharmaceutically acceptable adjuvants and vehicles which can be used in said vaccines are those adjuvants and vehicles known by the persons skilled in the art and normally used in the manufacture of vaccines.

In a particular embodiment, said vaccine is prepared in form of an aqueous solution or suspension in a pharmaceutically acceptable diluent, such as saline solution, phosphate-buffered saline solution (PBS), or any other pharmaceutically acceptable diluent.

The vaccine provided by this invention can be administered by any suitable administration route which results in a protective immune response against the heterologous sequence or epitope used, to which end said vaccine will be formulated in the dosage form suited to the chosen administration route. In a particular embodiment, the administration of the vaccine provided by this invention is carried out parenterally, for example, intraperitoneally, subcutaneously, etc.

The following Examples illustrate the invention and should not be considered limiting of the scope thereof. Example 1 clearly shows that the deletion of the C-terminal end of the IBDV VP3 protein hinders formation of IBDV VLPs, whereas Example 2 describes the generation of a recombinant baculovirus coexpressing the A1 and B1 open reading frames of the IBDV genome, and Example 3 illustrates obtaining whole IBDV VLPs from H5 cells infected with the recombinant baculovirus FBD/Poly-VP1. The materials and methods described below were used to implement the Examples.

### MATERIALS AND METHODS

**Cells and viruses.** The recombinant viruses VT7/VP3, VT7/PolyΔ907-1012, FB/Poly, FB/his-VP3 (wt), FB/his-VP3Δ253-257, FB/his-VP3Δ1-25, FB/his-VP3Δ26-52, FB/his-VP3Δ53-77, FB/his-VP3Δ78-100, FB/his-VP3Δ101-124, FB/his-VP3Δ125-150, FB/his-VP3Δ151-175, FB/his-VP3Δ176-200, FB/his-VP3Δ201-224 and FB/his-VP3Δ216-257 were disclosed previously (Fernández-Arias A et al. 1997. The major antigenic protein of infectious bursal disease virus, VP2, is an apoptotic inducer. J Virol. 71:8014-8; Kadono-Okuda K et al. 1995. Baculovirus-mediated production of the human growth hormone in larvae of the silkworm, Bombyx mori. Biochem Biophys Res Commun. 213:389-96; Kochan G et al. Characterization of the RNA binding activity of VP3, a major structural protein of IBDV. 2003. Archives of Virology 148:723-744; Martinez-Torrecuadrada JL et al. 2000. Different architectures in the assembly of infectious bursal disease virus capsid proteins expressed in insect cells. Virology 278:322-31).

The expression experiments were carried out with BSC-1 cells (American Type Culture Collection, ATCC; Catalogue CCL26), H5 [HighFive™ (GIBCO)] and Sf9 cells (GIBCO). The BSC-1 cells were cultured in Eagle modified Dulbecco medium supplemented with 10% fetal bovine serum. The 115 and Sf9 cells were cultured in TC-100 medium (GiBCO) supplemented with 10% fetal bovine serum. The viruses were amplified and titrated following previously disclosed protocols (Lombardo E et al. 2000. VP5, the nonstructural polypeptide of infectious bursal disease virus, accumulates within the host plasma membrane and induces cell lysis. Virology. 277:345-57; Martínez-Torrecuadrada JL ct al. 2000. Different architectures in the assembly of infectious bursal disease virus capsid proteins expressed in insect cells. Virology. 278:322-31).

The isolate of IBDV used was IBDV Soroa strain.

**Generation of recombinant baculoviruses.** The previously disclosed plasmid pFB/his-VP3 was used as a mold in the generation, by means of polymerase chain reaction (PCR), of the DNA fragments used in the construction of the plasmid vectors needed for the construction of the recombinant baculoviruses FB/his-VP3Δ248-257, FB/his-VP3Δ243-257, FB/his-VP3Δ238-257, FB/his-VP3Δ233-257, and FB/his-VP3Δ228-257. The PCR reactions were carried out using a common 5' primer (SEQ. ID. NO: 4) and 3' primer specific for each mutant (Table 1).

**Table 1 Generation of deletion mutants of the terminal carboxy end of His-VP3**

| **Mutant** | **Sequence** |
|---|---|
| His-VP3Δ248-257 | SEQ. ID. NO: 5 |
| His-VP3Δ243-257 | SEQ. ID. NO: 6 |
| His-VP3Δ238-257 | SEQ. ID. NO: 7 |
| His-VP3Δ233-257 | SEQ.ID.NO:8 |
| His-VP3Δ228-257 | SEQ. ID. NO: 9 |

After the PCR reactions, the corresponding DNA fragments were purified and digested with the restriction enzymes *Apa*I and *Kpn*I and ligated to the plasmid pFB/his-VP3 (Kochan G et al. 2003. Characterization of the RNA binding activity of VP3, a major structural protein of IBDV, Archives of Virology 148:723-744) previously digested with the same enzymes. The plasmid series generically referred to as pFB/his-ΔVP3 (pFB/his-VP3Δn-n' more specifically, wherein n and n' indicate the deleted region borders) containing deletions in the 5' end of the encoding region of VP3, were thus generated.

The construction of the plasmid vectors required for the generation of the recombinant baculoviruses FB/Poly△1008-1012, FB/PolyΔ1003-1012 and FB/PolyΔ998-1012 was carried out by means of the substitution of the *Xbα* I fragment (343 base pairs) with its homologues, containing the desired deletions, coming from the plasmids FB/his-VP3Δ233-257, FB/his-VP3Δ248-257, and FB/his-VP3Δ243-257, respectively.

The construction of the plasmid vector pFB/VP1 was carried out by means of cloning a DNA fragment, which contains the open reading frame of the gene of the IBDV VP1 protein, from the plasmid pBSKVP1 (Lombardo E et al. 1999. VP1, the putative RNA-dependent RNA polymerase of infectious bursal disease virus, forms complexes with the capsid protein VP3, leading to efficient encapsidation into virus-like particles. J. Virol. 73:6973-83) by means of digestion of the plasmid with the restriction enzyme *Cla*I, followed by treatment with the Klenow fragment of DNA polymerase I and subsequent treatment with the enzyme *Not*I. This fragment was subcloned into the vector pFastBacl (Invitrogen) previously digested with the restriction enzymes *Stu*I and *Not*I. The resulting plasmid was called pFB/VP1.

The plasmid vectors pFBD/his-VP3-VP1 and pFBD/Poly-VP1 were constructed by means of the insertion of the open reading frames of the genes of the VP3 and VP1 proteins in the vector pFastBacDual (Invitrogen). pFBD/VP 1 was generated by means of the insertion of a fragment containing the open reading frame of VP1 obtained by means of digestion with the enzyme *Not*I, followed by treatment with the Klenow fragment of DNA polymerase I and subsequent treatment with the enzyme *Xho*I, in the vector pFastBacDual (Invitrogen) previously digested with the enzymes *Xho*I and *Pvu*II. Then, the plasmid pFB/his-VP3 (Kochan G et al. 2003. Characterization of the RNA binding activity of VP3, a major structural protein of IBDV. Archives of Virology 148:723-744) was digested with the enzymes *Not*I and *Rsr*II, and the resulting fragment containing the open reading frame of his-VP3 was inserted in the plasmid pFBD/VP1 previously digested with the enzymes *Not*I and *Rsr*II. The resulting plasmid was called pFBD/his-VP3-VP1. Similarly, the open reading frame corresponding to the IBDV polyprotein was isolated from the plasmid pCIncoPoly (Maraver A et al. Identification and molecular characterization of the RNA polymerase-binding motif of the inner capsid protein VP3 of infectious bursal disease virus. J. Virol. 77:2459-2468) by means of digestion with the enzymes *Eco*RI and *Not*I*.* The corresponding DNA fragment was cloned into the plasmid pFBD/VP1 previously digested with the enzymes *Eco*RI and *Not*I, giving rise to the vector referred to as pFBD/Poly-VP1.

The recombinant baculoviruses described above were generated using the Bac-to-Bac system, following the protocols described by the manufacturer (Invitrogen).

**Purification by means of sucrose gradients and characterization of the structures derived from the expression of the IBDV polyprotein.** BSC-1 or H5 cells were infected with the described vaccine viruses or recombinant baculoviruses. The infected cells were harvested, lysed and processed as described above (Lombardo E et al. 1999. VP1, the putative RNA-dependent RNA polymerase of infectious bursal disease virus, forms complexes with the capsid protein VP3, leading to efficient encapsidation into virus-like particles. J. Virol. 73:6973-83; Castón JR et al. 2001. C terminus of infectious bursal disease virus major capsid protein VP2 is involved in definition of the number for capsid assembly. J. Virol. 75:10815-28).

**Electron microscopy.** Aliquots of 5 µl of the different fractions of the analyzed sucrose gradients were placed in electron microscopy grids. The samples thus prepared were negatively stained with a 2% uranyl acetate solution. The micrographs were obtained with a Jeol 1200 EXII microscope operating at 100 kV with magnifications of 20.000 or 40.000 X.

**Purification of his-VP3 fusion proteins and derivatives by means of immobilized metal affinity chromatography (IMAC).** H5 or Sf9 cells infected with the different recombinant viruses described were harvested at 72 h:p:i. Alter washing twice in phosphate buffered saline (PBS), the cells were resuspended in lysis buffer (Tris-HCl 50 mM, pH 8.0; NaCl 500 mM) supplemented with protease inhibitors (Complete Mini, Roche) and kept on ice for 20 minutes. Then the samples were subjected to centrifugation at 13,000 x g for 10 minutes at 4°C. The corresponding supernatants were subjected to purification by means of IMAC using a resin bound to cobalt (Talon, Clontech Laboratories, Inc., Palo Alto, CA) following the manufacturer instructions.

**Electrophoresis and Western blot.** The protein samples were resuspended in Laemmli buffer (King J & Laemmli UK. 1973. Bacteriophage T4 tail assembly: structural proteins and their genetic identification. J Mol Biol. 1973 Apr 5;75(2):315-37) and subjected to heating at 100°C for 5 minutes. The electrophoreses were carried out in 11% polyacrylamide gels. Then the proteins were transferred to nitrocellulose membranes by means of *electroblotting.* Prior to the incubation with specific autisera, the membranes were blocked by means of incubation for 1 hour at room temperature, with 5% powdered milk diluted in PBS.

**Immunofluorescence (IF) and confocal microscopy (CLSM)**. BSC-1 or H5 cells were grown on slide covers and infected with the recombinant baculoviruses or vaccine viruses. At the post-infection times indicated, the cells were washed two times with PBS and fixed with methanol at -20°C for 10 minutes. After the fixing, the slide covers were air dried, blocked in a 20% solution of recently born calf serum in PBS 45 minutes at room temperature and incubated with the indicated anti-sera. The samples were viewed by means of epifluorescence using a Zeiss Axiovert 200 microscope equipped with a Bio-Rad Radiance 2100 confocal system. The images were obtained using the Laser Sharp software package programs (Bio-Rad).

**Mass spectrophotometry (MS) analysis**. The proteins were passed through C-18 ZipTip tips minicolumns (Millipore, Bedford, MA, USA) and eluted in matrix solution (3,5-dimethoxy-4-hydroxycinnamic acid saturated in aqueous solution of 33% acetonitrile and 0.1% trifluoroacetic acid). An aliquot of 0.7 µl of the resulting mixture was placed in a steel MALDI probe which was subsequently air dried. The samples were analyzed using a Bruker Reflex™ IV MALDI-TOF mass spectrometer (Bruker-Franzen Analytic GmbH, Bremen, Germany) equipped with a SCOUT™ reflector source in positive ion reflector mode using delayed extraction. The acceleration voltage was 20 kV. The equipment was externally calibrated using mass signals corresponding to BSA and BSA dimers ranging from 20-130 m/z.

### EXAMPLE 1

### Deletion of the C-terminal end of the VP3 protein eliminates the formation of IBDV

### VLPs

It has recently been disclosed that the C-terminal end of VP3 contains the domain responsible for the interaction of this protein with the VP1 protein (Maraver A et al. Identification and molecular characterization of the RNA polymerase-binding motif of the inner capsid protein VP3 of infectious bursal disease virus). As a result, it was decided to analyze the possible role of the C-terminal region of VP3 in the morphogcncsis of IBDV VLPs. As a starting ground for this analysis, the recombinant vaccine virus referred to as VT7/Poly△907-1012, expressing a deleted form of VP3 lacking the 105 C-terminal end residues (SAnchez Martinez AB. 2000. "Caracterización de las modificacíones co y post-traduccionales de la poliproteína del virus de la bursitis infecciosa". Doctoral Thesis. Universidad Autónoma of Madrid. Facultad of Ciencias Biológicas), was used (Figure 1A). The SDS-PAGE and Western blot analysis showed that the deletion does not affect the cotranslational proteolytic processing of the polyprotein (Sánchez Martínez AB. 2000. Doctoral Thesis cited *supra*). Expression of the PolyΔ907-1012 protein gives rise to the formation of tubular structures similar to the type I tubules formed in cells infected with IBDV (Kaufer, I., and E. Weiss 1976. Electron-microscope studies on the pathogenesis of infectious bursal disease after intrabursal application of the causal virus. Avian Dis. 20:483-95). The tubular structures formed by expression of PolyΛ907-1012 were detected by means of immunofluorescence using antibodies anti-VPX/2 (anti-pVP2/VP2) and anti-VP3 (Figure 1B), and by means of electron microscopy of fractions obtained by means of purification on sucrose gradients (Figure 1C). The Western blot analysis confirmed the presence of VPX and VP3 in said tubules.

For the purpose of confirming that the mentioned phenotype was due to the deletion within the region corresponding to VP3, an experiment was carried out coinfecting BSC-1 cells with VT7/PolyΔ907-1012 and VT7/VP3. VT7/VP3 is a virus vaccine recombinant expressing the whole VP3 protein (Fernández-Arias A ct al. 1997. The major antigenic protein of infectious bursal disease virus, VP2, is an apoptotic inducer. J Virol. 71:8014-8). A confocal microscopy analysis showed that the coexpression of the whole VP3 protein produces a significant reduction in the formation of type I tubules. In the coinfected cells, the subcellular distribution of the VPX/VP3 proteins is characterized by the formation of short tubules and viroplasms similar to those detected in cells infected with the whole polyprotein (Figure 1B). This observation indicates that the coexpression of the whole VP3 protein partially salvages the ability of the PolyΔ907-1012 protein to form VLPs. The electron microscopy analysis of fractions derived from the coinfection confirmed this hypothesis. Therefore, the top fractions of the gradient were highly enriched in short tubules and quasi-spherical assemblies, called capsoids, with a diameter of 60-70 nm, together with a small proportion of VLPs of polygonal contour (Figure 1C). The Western blot analysis of the top fractions of the gradient, which contained the highest concentration of capsoids, clearly showed that they contained a larger ratio of whole VP3 protein than of VP3Δ907-1012 protein (data not shown). This result indicated that the incorporation of the whole VP3 protein in these structures is more efficient than that of the deleted form. These results show that the C-terminal end of VP3 plays a fundamental role in the morphogenesis of the IBDV capsid.

The VP3 protein undergoes a proteolytic processing in insect cells. It has previously been disclosed that the expression of the IBDV polyprotein in insect cells produces the assembly of long tubules formed by VPX trimer hexamers (Da Costa, B., C. Chevalier, C. Henry, J. C. Huet, S. Petit, J. Lcpault, H. Boot, and B. Delmas 2002. The capsid of infectious bursal disease virus contains several small peptides arising from the maturation process of pVP2. J Virol. 76:2393-402; Martínez-Torrecuadrada JL et al. 2000. Different architectures in the assembly of infectious bursal disease virus capsid proteins expressed in insect cells. Virology. 278:322-31). The similarity between the tubules observed in mammal cells infected with VT7/PolyΔ907-1012 and those detected in insect cells infected with recombinant baculoviruses expressing the whole polyprotein led to the analysis of the condition of the VP3 protein accumulated in insect cells. To that end, cell extracts infected with IBDV, VT7/Poly (Fernández-Arias A et al. 1998. Expression of ORF Al of infectious bursal disease virus results in the formation of virus-like particles. J. Gen. Virol. 79: 1047-54) and FB/Poly, respectively, were analyzed by means of Western blot using anti-VP3 serum (Fernández-Arias A et al. 1997. The major antigenic protein of infectious bursal disease virus, VP2, is an apoptotic inducer. J Virol. 71:8014-8). In cells infected with IBDV and VT7/Poly, the presence of a single band of 29 kDa, the expected: size of the whole VP3 protein, was detected by means of Western blot (Figure 2). On the contrary, in insect cells infected with FB/Poly, the presence of two bands corresponding to polypeptides of 29 and 27 kDa, respectively, was detected by means of Western blot (Figure 2). An analysis of the time expression showed that even though the appearance of the product of 27 kDa is slightly delayed with regard to the appearance of the product of 29 kDa, it becomes predominant in the later stage of infection (Figure 8A). A similar analysis carried out in Sf9 cells produced identical results (data not shown). These results show that in insect cells, the VP3 protein undergoes a post-translational modification giving rise to the accumulation of a product of 27 kDa.

The infection of insect cells with a recombinant baculovirus, FB/his-VP3, expressing a version of VP3 containing a six-histidine residue tag (6xhis), called his-VP3 (Figure 3A), gives rise to the accumulation of two molecular forms of the protein of 32 and 30 kDa, respectively, similar to those observed in cells infected with FB/Poly (Kochan G et al. 2003. Characterization of the RNA binding activity of VP3, a major structural protein of IBDV. Archives of Virology 148:723-744). Therefore, FB/his-VP3 was used as a tool to determine the origin of the smaller VP3 protein. To that end, both total cell extracts infected with FB/his-VP3 and protein purified by means of IMAC were analyzed by means of SDS-PAGE and Western blot using anti-VP3 serum (Figure 3B) and anti-6xhis (Figure 3C). As shown in Figure 3B, the polyprotein of the 30 kDa is present in the purified protein sample, which shows that its N-terminal end remains intact. On the other hand, both the product of 32 kDa and that of 30 kDa are recognized by both antisera (Figures 3B and 3C). These results strongly indicate that in insect cells, the VP3 protein undergoes proteolysis, giving rise to a product lacking a fragment of 2 kDa at its C-terminal end. For the purpose of firmly determining this possibility, six recombinant baculoviruses called his-VP3Δ253-257, his-VP3Δ248-257, his-VP3Δ243-257, his-VP3Δ238-257, his-VP3Δ233-257 and his-VP3Δ228-257, respectively (Figure 4A) were used [they correspond to those defined in the section of Materials and Methods, sub-section Cells and Virus, with an identical nomenclature, but preceded by "FB/" (indicative of the name of the plasmid used for generating the viruses (pFastBac1)]. These recombinant baculoviruses express a series of deletion forms of VP3 containing a histidine tag. The deletions were generated to progressively eliminate groups of 5 amino acid residues and thus generate a collection with growing deletions at the C-terminal end of the VP3 protein, as shown in Figure 4A. The expression of these proteins was analyzed by means of Western blot using anti-VP3 scrum. As shown in Figure 4B, the expression of the his-VP3 (his-VP3 wt) whole protein and of the his-VP3Δ253-257 mutant protein gave rise to the formation of doublets. On the other hand, the proteins containing deletions of 10 or more residues migrated according to their expected size, giving way to a single band (Figure 4B). This result shows that the C-terminal end of the VP3 protein is protcolytically processed and that the deletion of the cleavage site prevents proteolysis. The electrophoretic mobility of the his-VP3Δ248-257 protein is slightly less than that of the polypeptides generated by proteolytic processing of his-VP3 and his-VP3Δ253-257, which indicates that the processing occurs in the region located between residues 243 and 248. The C-terminal end of the his-VP3Δ248-257 protein is probably too short so as to allow the recognition on the part of the protease, and therefore it would not undergo proteolytic processing.

For the purpose of confirming the results obtained with the his-VP3 deletion mutants and precisely establishing the proteolytic cleavage site in the VP3 protein, H5 cell extracts infected with FB/his-VP3 were subjected to purification by means of IMAC. The resulting purified protein was analyzed by means of mass spectrometry. The experiment was repeated three times using independent purifications. The obtained results were similar in all cases (a difference in mass of less than 0.03%). Figure 5A shows the results of one of these experiments. The presence of two polypeptides of 32,004 and 30,444 Da, respectively, was determined. These results show that the proteolytic processing causes the elimination of a peptide of 1,560 Da from the C-terminal end of his-VP3. This size fits with the molecular mass (1,576 Da) corresponding to the 13 C-terminal residues of VP3 (SEQ. ID. NO: 3) (Figure 5B).

These results as a whole show that the VP3 protein is proteolytically processed in insect cells between the L244 and G245 residues, giving rise to a polypeptide lacking the 13 C-terminal residues.

### EXAMPLE 2

### Generation of a recombinant baculovirus coexpressing the A1 and B1 open reading frames of the IBDV genome

### 2.1 Construction of the plasmid pFBD/VP1

The nucleotide sequence corresponding to the B1 open reading frame of the IBDV genome was obtained from the plasmid pBSKVP1 described above (Lombardo E et al. 1999. VP1, the putative RNA-dependent RNA polymerase of infectious bursal disease virus, forms complexes with the capsid protein VP3, leading to efficient encapsidation into virus-like particles. J. Virol. 73:6973-83). The plasmid was purified and subjected to the following enzymatic treatments: i) digestion with the restriction enzyme *Not*I; ii) incubation with the Klenow fragment of DNA polymerase of *E. coli* in the presence of dNTPs; and iii) digestion with the restriction enzyme *Xho*I. Then the corresponding DNA fragment was purified and used for its cloning into the vector pFastBacDual (Invitrogen) previously treated with restriction enzymes *Xho*I and *Pvu*II. For this, the DNA fragment and the linearized plasmid were incubated in the presence of T4 DNA ligase to generate the plasmid pFBD/VP1.

### 2.2 Construction of the plasmid pFBD/Poly-VP1

The nucleotide sequence corresponding to the A1 open reading frame of the IBDV genome was obtained from the plasmid pCIneoPoly described above (Lombardo E et al. 1999. VP1, the putative RNA-dependent RNA polymerase of infectious bursal disease virus, forms complexes with the capsid protein VP3, leading to efficient encapsidation into virus-like particles. J. Virol. 73:6973-83). The plasmid was purified and incubated with the restriction enzymes *Eco*RI and *Not*I. The corresponding DNA fragment was purified and incubated with the plasmid pFBD/VP1, previously digested with the restriction enzymes *Eco*RI and *Not*I*,* in the presence of T4 DNA ligase to generate the plasmid pFBD/Poly-VP1. A bacteria culture transformed with said plasmid pFBD/Poly-VP1 has been deposited in the CECT with deposit number CECT 5777.

### 2.3 Obtaining the bacmid Bac/pFBD/Poly-VP1

This was carried out by means of the transformation of competent bacteria DH10Bac (Invitrogen), positive colony selection in selective medium and purification following the methodology disclosed by Invitrogen (catalog numbers 10359016 and 10608016).

### 2.4 Obtaining the recombinant baculovirus FBD/Poly-VP1

The virus was obtained by means of transfection of H5 cells (Invitrogen) with the bacmid Bac/pFBD/Poly-VP1 previously purified following the methodology disclosed by Invitrogen (catalog numbers 10359016 and 10608016).

### EXAMPLE 3

### Obtaining whole IBDV VLPs from H5 cells infected with the recombinant baculovirus FBD/PoLy-VP1

H5 cell cultures were infected with the recombinant virus FBD/Poly-VP1 (Example 2) using a multiplicity of infection of 5 plaque forming units per cell. The cultures were harvested at 72 hours post-infection (h.p.i). The cells were settled by means of centrifugation (1.500 x g for 10 minutes). The cellular sediment was resuspended in PES buffer (PIPES (1,4-piperazine ethanesulfonic acid) 25 mM, pH 6.2, NaCl 150 mM, CaCl₂ 20 mM). Then the cells were homogenized by means of three consecutive freezing/thawing cycles (-70°C/+37°C). The corresponding homogenate was centrifuged (10.000 x g for 15 minutes at 4°C). The resulting supernatant was harvested and used for the purification of the VLPs. To that end, a centrifuge tube with a 25% sucrose cushion (weight/volume), diluted in PES buffer of 4 ml, was prepared, depositing 8 ml of supernatant thereon. The tube was centrifuged (125.000 x g for 3 hours at 4°C). The resulting sediment was resuspended in 1 ml of PES buffer. Then a continuous 25-50% sucrose gradient in PES buffer was prepared in a centrifuge tube, depositing the resuspended sediment thereon. The tube was centrifuged (125.000 x g for 1 hour at 4°C). Then the gradient was fractioned into aliquots of 1 ml.

The different aliquots were analyzed by means of transmission electron microscopy. To that end, a volume of 5 µl of each sample was placed on a microscope grid. The samples were negatively stained with an aqueous solution of 2% uranyl acetate. A Jeol 1200 EXII microscope operating at 100 kV and at a nominal magnification of 40.000 X was used. This analysis showed the presence of whole VLPs structurally identical to the IBDV virions in the analyzed samples.

For the purpose of determining the protein composition of the VLPs detected by means of electron microscopy, the samples were analyzed by means of Western blot. To that end, the samples were subjected to polyacrylamide gel electrophoresis. The gels were subsequently transferred to nitrocellulose and incubated with anti-VPX/2 antibodies (anti-pVP2/VP2), anti-VP3 and anti-VP1. The results showed the presence of the VPX, VP2, VP3 and VP1 proteins in the fractions containing VLPs.

### MICROORGANISM DEPOSIT

A culture of the bacteria derived from DH5, carrier of a plasmid containing the TBDV polyprotein-VP1 genetic construction (pFBD/Poly-VP1), DH5-pFBD/poly-VP1, has been deposited in the Spanish Culture Type Collection (CECT), University of Valencia, Research Building, Burjasot Campus 46100 Burjasot Valencia, Spain, on March 8, 2003, with deposit number CECT 5777.

## Claims

1. A gene construct comprising (i) a nucleotide sequence comprising the open reading frames corresponding to the polyprotein of the infectious bursal disease virus (1BDV) operatively bound to a nucleotide sequence comprising a first promoter and (ii) a nucleotide sequence comprising the open reading frame corresponding to the IBDV VP1 protein operatively bound to a nucleotide sequence comprising a second promoter, wherein said first promoter is different from said second promoter.

2. A gene construct according to claim 1, wherein said first promoter is a viral promoter and said second promoter is a viral promoter different from said first promoter.

3. A gene construct according to claim 1 or 2, comprising:
(i) a nucleotide sequence comprising the open reading frames corresponding to the polyprotein IBDV operatively bound to a nucleotide sequence comprising a first promoter of a baculovirus, and
(ii) a nucleotide sequence comprising the open reading frame corresponding to the IBDV VP1 protein operatively bound to a nucleotide sequence comprising a second promoter of a baculovirus,
wherein said first baculovirus promoter is different from said second baculovirus promoter.

4. A gene construct according to claim 3, wherein said first baculovirus promoter is selected from the promoter of the p10 protein of the baculovirus *Autographa californica* nucleopolyhedrovirus (AcMNV) and the promoter of the polyhedrin of the baculovirus AcMNPV.

5. A gene construct according to claim 3, wherein said second baculovirus promoter is selected from the promoter of the AcMNPV p10 protein and the promoter of the AcMNPV polyhedrin.

6. A gene construct according to claim 3, wherein said first baculovirus promoter is the promoter of the AcMNPV p10 protein and said second baculovirus promoter is the promoter of the AcMNPV polyhcdrin; or wherein said first baculovirus promoter is the promoter of the AcMNPV polyhedrin and said second baculovirus promoter is the promoter of the AcMNPV p10 protein.

7. A gene construct according to any of claims 1 to 6, comprising the nucleotide sequence identified as SEQ. ID. NO: 1.

8. An expression system selected from:
a) an expression system comprising a gene construct according to any of claims 1 to 7, operatively bound to transcription, and optionally translation, control elements; and
b) an expression system comprising (1) a first gene construct, operatively bound to transcription, and optionally translation, control elements, wherein said first gene construct comprises a nucleotide sequence comprising the open reading frames corresponding to the IBDV polyprotein operatively bound to a nucleotide sequence comprising a first promoter, and (2) a second gene construct, operatively bound to transcription, and optionally translation, control elements, wherein said second gene construct comprises a nucleotide sequence comprising the open reading frame corresponding to the IBDV VP1 protein operatively bound to a nucleotide sequence comprising a second promoter.

9. An expression system according to claim 8, comprising a gene construct, operatively bound to transcription, and optionally translation, control elements, wherein said gene construct comprises (i) a nucleotide sequence comprising the open reading frames corresponding to the IBDV polyprotein operatively bound to a nucleotide sequence comprising a first baculovirus promoter and (ii) a nucleotide sequence comprising the open reading frame corresponding to the IBDV VP1 protein operatively bound to a nucleotide sequence comprising a second baculovirus promoter, wherein said first baculovirus promoter is different from said second baculovirus promoter.

10. An expression system according to claim 8, comprising (1) a first gene construct, operatively bound to transcription, and optionally translation, control elements, said first gene construct comprising a nucleotide sequence comprising the open reading frames corresponding to the IBDV polyprotein operatively bound to a nucleotide sequence comprising a first baculovirus promoter, and (2) a second gene construct, operatively bound to transcription, and optionally translation, control elements, said second gene construct comprising a nucleotide sequence comprising the open reading frame corresponding to the IBDV VP1 protein operatively bound to a nucleotide sequence comprising a second baculovirus promoter.

11. An expression system according to claim 10, wherein said first baculovirus promoter and said second baculovirus promoter are equal to or different from one another.

12. An expression system according to any of claims 8 to 11, selected from plasmids, bacmids, yeast artificial chromosomes (YACs), bacteria artificial chromosomes (BACs), P1 bacteriophage-based artificial chromosomes (PACs), cosmids and viruses, which can optionally contain a heterologous replication origin.

13. A host cell containing a gene construct according to any of claims 1 to 7, or an expression system according to any of claims 8 to 12.

14. A cell transformed, transfected or infected with an expression system according to any of claims 8 to 12.

15. A cell according to either of claims 13 or 14, selected from animal cells and bacteria.

16. A cell according to claim 15, **characterized in that** it is the bacteria identified as DH5-pFBD/Poly-VP1 which is deposited in the CECT with deposit number CECT 5777.

17. A cell according to claim 15, selected from insect cells, bird cells and mammal cells.

18. A dual recombinant baculovirus simultaneously expressing the IBDV polyprotein and the IBDV VP1 protein from (i) a nucleotide sequence comprising the open reading frames corresponding to the IBDV polyprotein operatively bound to a nucleotide sequence comprising a first baculovirus promoter, and from (ii) a nucleotide sequence comprising the open reading frame corresponding to the IBDV VP1 protein operatively bound to a nucleotide sequence comprising a second baculovirus promoter, wherein said first baculovirus promoter is different from said second baculovirus promoter.

19. The use of an expression system according to any of claims 8 to 12, or of a dual recombinant baculovims according to claim 17, for the production of whole empty viral capsids of IBDV.

20. A process for the production of whole empty viral capsids of the infectious bursal disease virus (IBDV) [whole IBDV VLPs] comprising culturing a host cell according to any of claims 13 to 17, and if desired, recovering said whole IBDV VLPs.

21. A process according to claim 20, wherein said host cell is a cell transformed, transfected or infected with an expression system comprising a gene construct comprising (i) a nucleotide sequence comprising the open reading frames corresponding to said IBDV polyprotein operatively bound to a nucleotide sequence comprising a first promoter and (ii) a nucleotide sequence comprising the open reading frame corresponding to said IBDV VP1 protein operatively bound to a nucleotide sequence comprising a second promoter, wherein said first promoter is different from said first promoter.

22. A process according to claim 20, wherein said host cell is a cell transformed, transfected or infected with an expression system comprising a gene-construct comprising (1) a first gene construct comprising a nucleotide sequence comprising the open reading frames corresponding to said IBDV polyprotein and (2) a second gene construct comprising a nucleotide sequence comprising the open reading frame corresponding to said 1BDV VP1 protein, each one of said nucleotide sequences comprising the ORFS corresponding to the viral polyprotein and to the IBDV VP1 protein being under the control of respective nucleotide sequences comprising respective promoters, equal to or different from one another.

23. A process according to either of claims 21 or 22, wherein said host cell is an insect cell.

24. A process according to claim 20, wherein said host cell is an insect cell, comprising the steps of:
a) preparing an expression system made up of a dual recombinant baculovirus comprising a gene construct comprising (i) a nucleotide sequence comprising the open reading frames corresponding to the IBDV polyprotein operatively bound to a nucleotide sequence comprising a first baculovirus promoter, said gene construct being operatively bound to transcription, and optionally translation, control elements, and (ii) a nucleotide sequence comprising the open reading frame corresponding to the IBDV VP1 protein operatively bound to a nucleotide sequence comprising a second baculovirus promoter, said gene construct being operatively bound to transcription, and optionally translation, control elements, wherein said baculovirus promoter is different from said second baculovirus promoter;
b) infecting insect cells with said expression system prepared in step a);
c) culturing the infected insect cells obtained in step b) under conditions allowing the expression of the recombinant proteins and their assembly to form whole IBDV VLPs; and
d) if desired, isolating and optionally purifying said whole IBDV VLPs.

25. A process according to claim 20, wherein said host cell is an insect cell, comprising the steps of:
a) preparing an expression system made up of (1) a first recombinant baculovirus comprising a gene construct comprising a nucleotide sequence comprising the open reading frames corresponding to the IBDV polyprotein operatively bound to a baculovirus promoter, said gene construct being operatively bound to transcription, and optionally translation, control elements, and of (2) a second recombinant baculovirus comprising a gene construct comprising a nucleotide sequence comprising the open reading frame corresponding to the IBDV VP1 protein operatively bound to a promoter of a baculovirus, said gene construct being operatively bound to transcription, and optionally translation, control elements;
b) infecting insect cells with said expression system prepared in step a);
c) culturing the infected insect cells obtained in step b) under conditions allowing the expression of the recombinant proteins and their assembly to form whole IBDV VLPs; and
d) if desired, isolating and optionally purifying said whole TBDV VLPs.

26. Whole empty capsids of the infectious bursal disease virus (IBDV) [whole IBDV VLPs], obtained according to the process of any of claims 20 to 25.

27. Whole empty capsids of the infectious bursal disease virus (IBDV) [whole IBDV VLPs], **characterized by** containing the VPX, VP2, VP3 and VP1 proteins of IBDV.

28. The use of whole empty capsids of the infectious bursal disease virus (IBDV) [whole IBDV VLPs], according to either of claims 26 or 27, in the manufacture of a medicament.

29. The use according to claim 28, wherein said medicament is a vaccine against the avian disease called infectious bursal disease.

30. The use according to claim 28, wherein said medicament is a gene therapy vector,

31. A vaccine comprising a therapeutically effective amount of whole empty capsids of IBDV [whole IBDV VLPs], according to either of claims 26 or 27, optionally combined with one or more pharmaceutically acceptable adjuvants and/or vehicles.

32. A vaccine according to claim 31, for protecting birds from the infection caused by IBDV.

33. A vaccine according to claim 32, wherein said birds are selected from the group formed by chickens, turkeys, geese, ganders, pheasants, partridges and ostriches.

34. A vaccine for protecting chickens from the infection caused by the infectious bursal disease virus (IBDV), comprising a therapeutically effective amount of whole empty capsids of IBDV, whole IBDV VLPs, according to either of claims 26 or 27, optionally combined with one or more pharmaceutically acceptable adjuvants and/or vehicles.

35. A process for obtaining a dual recombinant baculovirus allowing the simultaneous expression in insect cells of the polyprotein of the infectious bursal disease virus (IBDV) and of the IBDV VP 1 protein from two independent open reading frames and each one of them controlled by a different baculovirus promoter, comprising:
a) constructing a plasmid carrying a gene construct containing (i) a nucleotide sequence comprising the open reading frames corresponding to the IBDV polyprotein operatively bound to a nucleotide sequence comprising a first promoter of a baculovirus, and (ii) a nucleotide sequence comprising the open reading frame corresponding to the IBDV VP1 protein operatively bound to a nucleotide sequence comprising a second promoter of a baculovirus, wherein said first baculovirus promoter is different from said second baculovirus promoter;
b) obtaining a recombinant bacmid, allowing the simultaneous expression during its replicative cycle of the polyprotein and the IBDV VP1 protein under transcriptional control of said baculovirus promoters, by means of the transformation of competent bacteria with the plasmid obtained in a); and
c) obtaining a dual recombinant baculovirus, allowing the simultaneous expression of the open reading frames corresponding to the polyprotein and the IBDV VP1 protein under transcriptional control of said baculovirus promoters, by means of transformation of insect cells with the recombinant bacmid of b).

36. A process according to claim 35, wherein:
- said first baculovirus promoter is the promoter of the AcMNV p10 protein and said second baculovirus promoter is the promoter of the AcMNPV polyhedrin, or vice versa;
- the plasmid obtained in a) is the one identified as pFBD/Poly-VP1;
- the competent bacteria of b) are *E*. *coli* DH10Bac;
- the recombinant bacmid obtained in b) is the one identified as Bac/pFBD/Poly-VP1; and
- the recombinant baculovirus obtained is the one identified as FBD/Poly-VP1.

37. A process according to claim 35 or 36, further comprising the infection of insect cells with the dual recombinant baculovirus obtained in step c).

38. A process according to claim 37, wherein said insect cells are H5 or *Spodoptera frugiperda* S19 cells.


SEQUENCE LISTING

<110> CONSEJO SUPERIOR DE INVESTIGACIONES CIENTÍFICAS
<110> BIONOSTRA, S.L.

<120> WHOLE EMPTY VIRAL PARTICLES OF THE INFECTIOUS BURSAL DISEASE
VIRUS (IBDV), PRODUCTION PROCESS AND APPLICATIONS

<150> P200300751
<151> 2003-03-31

<160> 9

<170> PatentIn version 3.1

<210> 1
<211> 10909
<212> DNA
<213> Artificial sequence

<220>
<223> IBDV polyprotein-VP1 gene construction

<220>
<221> gene
<222> (3)..(3041)
<223> Open reading frame of IBDV polyprotein in reverse complementary
strand

<220>
<221> promoter
<222> (3083)..(3211)
<223> AcMNV polyhedrin promoter

<220>
<221> promoter
<222> (3230)..(3351)
<223> AcMNV p10 promoter

<220>
<221> CDS
<222> (3388)..(6027)
<223> Open reading frame of IBDV VP1 protein

<220>
<221> polyA_site
<222> (6068)..(6331)
<223>

<220>
<221> gene
<222> (6901)..(7434)
<223> Gentamicin resistance gene

<220>
<221> misc_feature
<222> (7501)..(7725)
<223> Minitransposon Tn7R

<220>
<221> gene

<222>  (8787)..(9647)
<223>  Ampicillin resistance gene

<220>
<221>  misc_feature
<222>  (9854)..(10234)
<223>  F1

<220>
<221>  misc_feature
<222>  (10418)..(10583)
<223>  Minitransposon Tn7L

<400>  1
gctcactcaa ggtcctcatc agagacggtc ctgatccagc ggcccagccg accagggggt      60

ctctgtgttg gagcattggg ttttggcttg ggctttggta gagcccgcct gggattgcga     120

tgcttcatct ccatcgcagt caagagcaga tctttcatct gttcttggtt tgggccacgt     180

ccatggttga tttcatagac tttggcaact tcgtctatga aagcttgggg tggctctgcc     240

tgtcctggag ccccgtagat cgacgtagct gcccttagga tttgttcttc tgatgccaac     300

cggctcttct ctgcatgcac gtagtctaga tagtcctcgt ttgggtccgg tatttctcgt     360

ttgttctgcc agtactttac ctggcctggg cttggccctc ggtgcccatt gagtgctacc     420

cattctggtg ttgcaaagta gatgcccatg gtctccatct tctttgagat ccgtgtgtct     480

ttttccctct gtgcttcctc tggtgtgggg ccccgagcct ccactccgta gcctgctgtc     540

ccgtacttgg ccctttgcga cttgctgcct gcttgtggtg cgtttgcaag aaaatttcgc     600

atccgatggg cgttcgggtc gctgagtgcg aagttggcca tgtcagtcac aatcccattc     660

tcttccagcc acatgaacac actgagtgca gattggaata gtgggtccac gttggctgct     720

gcttccattg ctctgacggc actctcgagt tcgggggtct ctttgaactc tgatgcagcc     780

atggcaaggt ggtactggcg tcctgcattg ggtggaaggt atggtaggtt gaggtagggg     840

agcctgtccc agtcgcgtgg attgtgaggg aaacgtttga tgaacgttgc ccagttgggc     900

ccggtgttta catcgaatgc tccgggacca gccaacctaa ggccaagtcg gtgtgcagta     960

gcgagcttgg tgcttctaaa gcttactttc tcaatctcgc cacaagcatt gagggctccc    1020

gtcatagcca catggattgg gactttgggt cgaaacacat ccatgtaagc tatggctaga    1080

tttccactgt ttcccacaat aggaggtatg ggatctttgg acagcataat gctgtcgtcc    1140

cagacatcat ctattgggac aacggtgtag tctctcccag tctccagtgg aagtacccca    1200

tctggagcat atccatagac tctgtgtcca gagagagttc gtatgaagga tcctctttga    1260

gatggaggtt ggaggtcttc tcgcacgcct tcaatgacag caaacatttt gctgttcaat    1320

gctttgggtg tcatggcgtc ttccactgtc gtaataacca cagggaatag cgtggcaccc    1380

tctcttaaca cgcagtcgag gttgtgtgca ccgcggagta ccccaggtga agcaagaatc    1440

ccgtcgacta cgggattctg gggcacctgg aatagattcg cgactacctc gtaccccttg 1500
tcggcggcga gagtcagctg ccttatgcgg cctgaggcag ctcttgcttt tcctgacgcg 1560
gctcgagcag ttcctgaagc ggcctgggcc tcatcgccca gcaggtagtc tacaccttcc 1620
ccaattgcat gggctagggg agcggcaggt gggaacaatg tggagaccac cggcacagct 1680
atcctcctta tggcccggat tatgtctttg aagccgaatg ctcctgcaat cttcagggga 1740
gagttgaggt cggccacctc catgaagtat tcacgaaagt cagtgtactc ccttgttggc 1800
cagacggtct tgatgccaag acggtccctc tcactcagta tcaattttgt gtagttcatg 1860
gctcctgggt caaatcggcc gtattctgta accaggttct ttgctagttc aggatttggg 1920
atcagctcga agttgctcac cccagcgacc gtaacgacgg atcctgttgc cactctttcg 1980
taggccacta gcgtgacggg acggagggcc cctggatagt tgccaccatg gatcgtcact 2040
gctaggctcc ctcttgccga ccatgacatc tgatcccctg cctgaccacc acttttggag 2100
gtcactatct ccagtttgat ggatgtgatt ggctgggtta tctcgtttgt tggaatcaca 2160
agattgaatg gcataaggtt gtcggtgccg gtcgtcagcc cattgtttgc ggccacagcc 2220
ctggtgatta ccgttgtccc atcaaagcct atgaggtaga tggtggcgcc cagtacaagg 2280
ccgtggacgc ttgttcgaaa cacgagctct cccccaacgc tgaggcttgt gatggcatca 2340
atgttggctg agaacagtgt gattgttacc ccacctggtt ggtactgtga tgagaattgg 2400
taatcatcgg ctgcagttat ggtgtagact ctgggcctgt cactgctgtc acatgtggct 2460
accatttttg ggtcaagccc tattgcggga atggggtcac caagcctcac atacccaaga 2520
tcatatgatg tgggtaagct gaggacggtg accccttccc ctactaggac gttcccaatt 2580
ttgtcgttga tgttggctgt tgcagacatc aacccattgt agctaacatc tgtcagttca 2640
ctcaggcttc cttggaaggt cacggcgttt atggtgccgt ttagtgcata aacgccacca 2700
ggaagtgtgc ttgacctcac tgtgagactc cgactcacta gcctgcagta gttgtaactg 2760
gccggtaggt tctgggcagt caggagcatc tgatcgaact tgtagttccc attgccctgc 2820
agtgtgtagt gagcacccac aattgagcca gggaatccag ggaaaaagac aattagccct 2880
gaccctgtgt cccccacagt caaattgtag gtcgaggtct ctgacctgag agtgtgcttc 2940
tccagggtgt cgtccggaat ggacgccggt ccggttgttg gcatcagaag gctccgtatg 3000
aacggaacaa tctgctgggt ttgatctgac aggtttgtca tcgatgcgat cgaattccgc 3060
gcgcttcgga ccgggatccg cgcccgatgg tgggacggta tgaataatcc ggaatattta 3120
taggtttttt tattacaaaa ctgttacgaa aacagtaaaa tacttattta tttgcgagat 3180
ggttatcatt ttaattatct ccatgatcta ttaatattcc ggagtatacg gacctttaat 3240

tcaacccaac acaatatatt atagttaaat aagaattatt atcaaatcat ttgtatatta 3300

attaaaatac tatactgtaa attacatttt atttacaatc actcgacgaa gacttgatca 3360

cccgggatct cgaggtcgac ggtatcg atg agt gac gtt ttc aat agt cca cag 3414
Met Ser Asp Val Phe Asn Ser Pro Gln
1               5

gcg cga agc acg atc tca gca gcg ttc ggc ata aag cct act gct gga 3462
Ala Arg Ser Thr Ile Ser Ala Ala Phe Gly Ile Lys Pro Thr Ala Gly
10                  15                  20                  25

caa gac gtg gaa gaa ctc ttg atc cct aaa gtt tgg gtg cca cct gag 3510
Gln Asp Val Glu Glu Leu Leu Ile Pro Lys Val Trp Val Pro Pro Glu
                30                  35                  40

gat ccg ctt gcc agc cct agt cga ctg gca aag ttc ctc aga gag aac 3558
Asp Pro Leu Ala Ser Pro Ser Arg Leu Ala Lys Phe Leu Arg Glu Asn
            45                  50                  55

ggc tac aaa gtt ttg cag ccg cgg tct ctg ccc gag aat gag gag tat 3606
Gly Tyr Lys Val Leu Gln Pro Arg Ser Leu Pro Glu Asn Glu Glu Tyr
        60                  65                  70

gag acc gac caa ata ctc cca gac tta gca tgg atg cga cag ata gaa 3654
Glu Thr Asp Gln Ile Leu Pro Asp Leu Ala Trp Met Arg Gln Ile Glu
    75                  80                  85

ggg gct gtt tta aaa ccc act cta tct ctc cct att gga gat cag gag 3702
Gly Ala Val Leu Lys Pro Thr Leu Ser Leu Pro Ile Gly Asp Gln Glu
90                  95                  100                 105

tac ttc cca aag tac tac cca aca cat cgc cct agc aag gag aag ccc 3750
Tyr Phe Pro Lys Tyr Tyr Pro Thr His Arg Pro Ser Lys Glu Lys Pro
                110                 115                 120

aat gcg tac cca cca gac atc gca cta ctc aag cag atg att tac ctg 3798
Asn Ala Tyr Pro Pro Asp Ile Ala Leu Leu Lys Gln Met Ile Tyr Leu
            125                 130                 135

ttt ctc cag gtt cca gag gcc aac gag ggc cta aag gat gaa gta acc 3846
Phe Leu Gln Val Pro Glu Ala Asn Glu Gly Leu Lys Asp Glu Val Thr
        140                 145                 150

ctc ttg acc caa aac ata agg gac aag gcc tat gga agt ggg acc tac 3894
Leu Leu Thr Gln Asn Ile Arg Asp Lys Ala Tyr Gly Ser Gly Thr Tyr
    155                 160                 165

atg gga caa gca aat cga ctt gtg gcc atg aag gag gtc gcc act gga 3942
Met Gly Gln Ala Asn Arg Leu Val Ala Met Lys Glu Val Ala Thr Gly
170                 175                 180                 185

aga aac cca aac aag gat cct cta aag ctt ggg tac act ttt gag agc 3990
Arg Asn Pro Asn Lys Asp Pro Leu Lys Leu Gly Tyr Thr Phe Glu Ser
                190                 195                 200

atc gcg cag cta ctt gac atc aca cta ccg gta ggc cca ccc ggt gag 4038
Ile Ala Gln Leu Leu Asp Ile Thr Leu Pro Val Gly Pro Pro Gly Glu
            205                 210                 215

gat gac aag ccc tgg gtg cca ctc aca aga gtg ccg tca cgg atg ttg 4086

Asp Asp Lys Pro Trp Val Pro Leu Thr Arg Val Pro Ser Arg Met Leu
        220                 225                 230

gtg ctg acg gga gac gta gat ggc gac ttt gag gtt gaa gat tac ctt      4134
Val Leu Thr Gly Asp Val Asp Gly Asp Phe Glu Val Glu Asp Tyr Leu
    235                 240                 245

ccc aaa atc aac ctc aag tca tca agt gga cta cca tat gta ggt cgc      4182
Pro Lys Ile Asn Leu Lys Ser Ser Ser Gly Leu Pro Tyr Val Gly Arg
250                 255                 260                 265

acc aaa gga gag aca att ggc gag atg ata gct ata tca aac cag ttt      4230
Thr Lys Gly Glu Thr Ile Gly Glu Met Ile Ala Ile Ser Asn Gln Phe
                270                 275                 280

ctc aga gag cta tca aca ctg ttg aag caa ggt gca ggg aca aag ggg      4278
Leu Arg Glu Leu Ser Thr Leu Leu Lys Gln Gly Ala Gly Thr Lys Gly
            285                 290                 295

tca aac aag aag aag cta ctc agc atg tta agt gac tat tgg tac tta      4326
Ser Asn Lys Lys Lys Leu Leu Ser Met Leu Ser Asp Tyr Trp Tyr Leu
        300                 305                 310

tca tgc ggg ctt ttg ttt cca aag gct gaa agg tac gac aaa agt aca      4374
Ser Cys Gly Leu Leu Phe Pro Lys Ala Glu Arg Tyr Asp Lys Ser Thr
    315                 320                 325

tgg ctc acc aag acc cgg aac ata tgg tca gct cca tcc cca aca cac      4422
Trp Leu Thr Lys Thr Arg Asn Ile Trp Ser Ala Pro Ser Pro Thr His
330                 335                 340                 345

ctc atg atc tcc atg atc acc tgg ccc gtg atg tcc aac agc cca aat      4470
Leu Met Ile Ser Met Ile Thr Trp Pro Val Met Ser Asn Ser Pro Asn
                350                 355                 360

aac gtg ttg aac att gaa ggg tgt cca tca ctc tac aaa ttc aac ccg      4518
Asn Val Leu Asn Ile Glu Gly Cys Pro Ser Leu Tyr Lys Phe Asn Pro
            365                 370                 375

ttc aga gga ggg ttg aac agg atc gtc gag tgg ata ttg gcc ccg gaa      4566
Phe Arg Gly Gly Leu Asn Arg Ile Val Glu Trp Ile Leu Ala Pro Glu
        380                 385                 390

gaa ccc aag gct ctt gta tat gcg gac aac ata tac att gtc cac tca      4614
Glu Pro Lys Ala Leu Val Tyr Ala Asp Asn Ile Tyr Ile Val His Ser
    395                 400                 405

aac acg tgg tac tca att gac cta gag aag ggt gag gca aac tgc act      4662
Asn Thr Trp Tyr Ser Ile Asp Leu Glu Lys Gly Glu Ala Asn Cys Thr
410                 415                 420                 425

cgc caa cac atg caa gcc gca atg tac tac ata ctc acc aga ggg tgg      4710
Arg Gln His Met Gln Ala Ala Met Tyr Tyr Ile Leu Thr Arg Gly Trp
                430                 435                 440

tca gac aac ggc gac cca atg ttc aat caa aca tgg gcc acc ttt gcc      4758
Ser Asp Asn Gly Asp Pro Met Phe Asn Gln Thr Trp Ala Thr Phe Ala
            445                 450                 455

atg aac att gcc cct gct cta gtg gtg gac tca tcg tgc ctg ata atg 4806
Met Asn Ile Ala Pro Ala Leu Val Val Asp Ser Ser Cys Leu Ile Met
        460                 465                 470

aac ctg caa att aag acc tat ggt caa ggc agc ggg aat gca gcc acg 4854
Asn Leu Gln Ile Lys Thr Tyr Gly Gln Gly Ser Gly Asn Ala Ala Thr
    475                 480                 485

ttc atc aac aac cac ctc ttg agc acg cta gtg ctt gac cag tgg aac 4902
Phe Ile Asn Asn His Leu Leu Ser Thr Leu Val Leu Asp Gln Trp Asn
490                 495                 500                 505

ttg atg aga cag ccc aga cca gac agc gag gag ttc aaa tca att gag 4950
Leu Met Arg Gln Pro Arg Pro Asp Ser Glu Glu Phe Lys Ser Ile Glu
                510                 515                 520

gac aag cta ggt atc aac ttt aag att gag agg tcc att gat gat atc 4998
Asp Lys Leu Gly Ile Asn Phe Lys Ile Glu Arg Ser Ile Asp Asp Ile
            525                 530                 535

agg ggc aag ctg aga cag ctt gtc ctc ctt gca caa cca ggg tac ctg 5046
Arg Gly Lys Leu Arg Gln Leu Val Leu Leu Ala Gln Pro Gly Tyr Leu
        540                 545                 550

agt ggg ggg gtt gaa cca gaa caa tcc agc cca act gtt gag ctt gac 5094
Ser Gly Gly Val Glu Pro Glu Gln Ser Ser Pro Thr Val Glu Leu Asp
    555                 560                 565

cta cta ggg tgg tca gct aca tac agc aaa gat ctc ggg atc tat gtg 5142
Leu Leu Gly Trp Ser Ala Thr Tyr Ser Lys Asp Leu Gly Ile Tyr Val
570                 575                 580                 585

ccg gtg ctt gac aag gaa cgc cta ttt tgt tct gct gcg tat ccc aag 5190
Pro Val Leu Asp Lys Glu Arg Leu Phe Cys Ser Ala Ala Tyr Pro Lys
                590                 595                 600

gga gta gag aac aag agt ctc aag tcc aaa gtc ggg atc gag cag gca 5238
Gly Val Glu Asn Lys Ser Leu Lys Ser Lys Val Gly Ile Glu Gln Ala
            605                 610                 615

tac aag gta gtc agg tat gag gcg tta agg ttg gta ggt ggt tgg aac 5286
Tyr Lys Val Val Arg Tyr Glu Ala Leu Arg Leu Val Gly Gly Trp Asn
        620                 625                 630

tac cca ctc ctg aac aaa gcc tgc aag aat aac gca ggc gcc gct cgg 5334
Tyr Pro Leu Leu Asn Lys Ala Cys Lys Asn Asn Ala Gly Ala Ala Arg
    635                 640                 645

cgg cat ctg gag gcc aag ggg ttc cca ctc gac gag ttc cta gcc gag 5382
Arg His Leu Glu Ala Lys Gly Phe Pro Leu Asp Glu Phe Leu Ala Glu
650                 655                 660                 665

tgg tct gag ctg tca gag ttc ggt gag gcc ttc gaa ggc ttc aat atc 5430
Trp Ser Glu Leu Ser Glu Phe Gly Glu Ala Phe Glu Gly Phe Asn Ile
                670                 675                 680

aag ctg acc gta aca tct gag agc cta gcc gaa ctg aac aag cca gta 5478
Lys Leu Thr Val Thr Ser Glu Ser Leu Ala Glu Leu Asn Lys Pro Val
            685                 690                 695

ccc ccc aag ccc cca aat gtc aac aga cca gtc aac act ggg gga ctc 5526
Pro Pro Lys Pro Pro Asn Val Asn Arg Pro Val Asn Thr Gly Gly Leu
        700                 705                 710

aag gca gtc agc aac gcc ctc aag acc ggt cgg tac agg aac gaa gcc 5574
Lys Ala Val Ser Asn Ala Leu Lys Thr Gly Arg Tyr Arg Asn Glu Ala
    715                 720                 725

gga ctg agt ggt ctc gtc ctt cta gcc aca gca aga agc cgt ctg caa 5622
Gly Leu Ser Gly Leu Val Leu Leu Ala Thr Ala Arg Ser Arg Leu Gln
730                 735                 740                 745

gat gca gtt aag gcc aag gca gaa gcc gag aaa ctc cac aag tcc aag 5670
Asp Ala Val Lys Ala Lys Ala Glu Ala Glu Lys Leu His Lys Ser Lys
                750                 755                 760

cca gac gac ccc gat gca gac tgg ttc gaa aga tca gaa act ctg tca 5718
Pro Asp Asp Pro Asp Ala Asp Trp Phe Glu Arg Ser Glu Thr Leu Ser
            765                 770                 775

gac ctt ctg gag aaa gcc gac atc gcc agc aag gtc gcc cac tca gca 5766
Asp Leu Leu Glu Lys Ala Asp Ile Ala Ser Lys Val Ala His Ser Ala
        780                 785                 790

ctc gtg gaa aca agc gac gcc ctt gaa gca gtt cag tcg act tcc gtg 5814
Leu Val Glu Thr Ser Asp Ala Leu Glu Ala Val Gln Ser Thr Ser Val
    795                 800                 805

tac acc ccc aag tac cca gaa gtc aag aac cca cag acc gcc tcc aac 5862
Tyr Thr Pro Lys Tyr Pro Glu Val Lys Asn Pro Gln Thr Ala Ser Asn
810                 815                 820                 825

ccg gtt gtt ggg ctc cac ctg ccc gcc aag agg gcc acc ggt gtc cag 5910
Pro Val Val Gly Leu His Leu Pro Ala Lys Arg Ala Thr Gly Val Gln
                830                 835                 840

gcc gct ctt ctc gga gca gga acg agc aga cca atg ggg atg gag gcc 5958
Ala Ala Leu Leu Gly Ala Gly Thr Ser Arg Pro Met Gly Met Glu Ala
            845                 850                 855

cca aca cgg tcc aag aac gcc gtg aaa atg gcc aaa cgg cgg caa cgc 6006
Pro Thr Arg Ser Lys Asn Ala Val Lys Met Ala Lys Arg Arg Gln Arg
        860                 865                 870

caa aag gag agc cgc caa tag ccatgaggcg gccctgatgc atagcatgcg 6057
Gln Lys Glu Ser Arg Gln
    875

gtaccgggag atgggggagg ctaactgaaa cacggaagga gacaataccg gaaggaaccc 6117

gcgctatgac ggcaataaaa agacagaata aaacgcacgg gtgttgggtc gtttgttcat 6177

aaacgcgggg ttcggtccca gggctggcac tctgtcgata ccccaccgag accccattgg 6237

gaccaatacg cccgcgtttc ttccttttcc ccaccccaac ccccaagttc gggtgaaggc 6297

ccagggctcg cagccaacgt cggggcggca agccctgcca tagccactac gggtacgtag 6357

gccaaccact agaactatag ctagagtcct gggcgaacaa acgatgctcg ccttccagaa 6417

aaccgaggat gcgaaccact tcatccgggg tcagcaccac cggcaagcgc cgcgacggcc 6477

gaggtctacc gatctcctga agccagggca gatccgtgca cagcaccttg ccgtagaaga 6537

acagcaaggc cgccaatgcc tgacgatgcg tggagaccga aaccttgcgc tcgttcgcca 6597

gccaggacag aaatgcctcg acttcgctgc tgcccaaggt tgccgggtga cgcacaccgt 6657

ggaaacggat gaaggcacga acccagttga cataagcctg ttcggttcgt aaactgtaat 6717

gcaagtagcg tatgcgctca cgcaactggt ccagaacctt gaccgaacgc agcggtggta 6777

acggcgcagt ggcggttttc atggcttgtt atgactgttt ttttgtacag tctatgcctc 6837

gggcatccaa gcagcaagcg cgttacgccg tgggtcgatg tttgatgtta tggagcagca 6897

acgatgttac gcagcagcaa cgatgttacg cagcagggca gtcgccctaa aacaaagtta 6957

ggtggctcaa gtatgggcat cattcgcaca tgtaggctcg gccctgacca agtcaaatcc 7017

atgcgggctg ctcttgatct tttcggtcgt gagttcggag acgtagccac ctactcccaa 7077

catcagccgg actccgatta cctcgggaac ttgctccgta gtaagacatt catcgcgctt 7137

gctgccttcg accaagaagc ggttgttggc gctctcgcgg cttacgttct gcccaggttt 7197

gagcagccgc gtagtgagat ctatatctat gatctcgcag tctccggcga gcaccggagg 7257

cagggcattg ccaccgcgct catcaatctc ctcaagcatg aggccaacgc gcttggtgct 7317

tatgtgatct acgtgcaagc agattacggt gacgatcccg cagtggctct ctatacaaag 7377

ttgggcatac gggaagaagt gatgcacttt gatatcgacc caagtaccgc cacctaacaa 7437

ttcgttcaag ccgagatcgg cttcccggcc gcggagttgt tcggtaaatt gtcacaacgc 7497

cgcgaatata gtctttacca tgcccttggc cacgcccctc tttaatacga cgggcaattt 7557

gcacttcaga aaatgaagag tttgctttag ccataacaaa agtccagtat gctttttcac 7617

agcataactg gactgatttc agtttacaac tattctgtct agtttaagac tttattgtca 7677

tagtttagat ctattttgtt cagtttaaga ctttattgtc cgcccacacc cgcttacgca 7737

gggcatccat ttattactca accgtaaccg attttgccag gttacgcggc tggtctgcgg 7797

tgtgaaatac cgcacagatg cgtaaggaga aaataccgca tcaggcgctc ttccgcttcc 7857

tcgctcactg actcgctgcg ctcggtcgtt cggctgcggc gagcggtatc agctcactca 7917

aaggcggtaa tacggttatc cacagaatca ggggataacg caggaaagaa catgtgagca 7977

aaaggccagc aaaaggccag gaaccgtaaa aaggccgcgt tgctggcgtt tttccatagg 8037

ctccgccccc ctgacgagca tcacaaaaat cgacgctcaa gtcagaggtg gcgaaacccg 8097

acaggactat aaagatacca ggcgtttccc cctggaagct ccctcgtgcg ctctcctgtt 8157

ccgaccctgc cgcttaccgg atacctgtcc gcctttctcc cttcgggaag cgtggcgctt 8217

tctcaatgct cacgctgtag gtatctcagt tcggtgtagg tcgttcgctc caagctgggc 8277
tgtgtgcacg aaccccccgt tcagcccgac cgctgcgcct tatccggtaa ctatcgtctt 8337
gagtccaacc cggtaagaca cgacttatcg ccactggcag cagccactgg taacaggatt 8397
agcagagcga ggtatgtagg cggtgctaca gagttcttga agtggtggcc taactacggc 8457
tacactagaa ggacagtatt tggtatctgc gctctgctga agccagttac cttcggaaaa 8517
agagttggta gctcttgatc cggcaaacaa accaccgctg gtagcggtgg tttttttgtt 8577
tgcaagcagc agattacgcg cagaaaaaaa ggatctcaag aagatccttt gatcttttct 8637
acggggtctg acgctcagtg gaacgaaaac tcacgttaag ggattttggt catgagatta 8697
tcaaaaagga tcttcaccta gatcctttta aattaaaaat gaagttttaa atcaatctaa 8757
agtatatatg agtaaacttg gtctgacagt taccaatgct taatcagtga ggcacctatc 8817
tcagcgatct gtctatttcg ttcatccata gttgcctgac tccccgtcgt gtagataact 8877
acgatacggg agggcttacc atctggcccc agtgctgcaa tgataccgcg agacccacgc 8937
tcaccggctc cagatttatc agcaataaac cagccagccg gaagggccga gcgcagaagt 8997
ggtcctgcaa ctttatccgc ctccatccag tctattaatt gttgccggga agctagagta 9057
agtagttcgc cagttaatag tttgcgcaac gttgttgcca ttgctacagg catcgtggtg 9117
tcacgctcgt cgtttggtat ggcttcattc agctccggtt cccaacgatc aaggcgagtt 9177
acatgatccc ccatgttgtg caaaaaagcg gttagctcct tcggtcctcc gatcgttgtc 9237
agaagtaagt tggccgcagt gttatcactc atggttatgg cagcactgca taattctctt 9297
actgtcatgc catccgtaag atgcttttct gtgactggtg agtactcaac caagtcattc 9357
tgagaatagt gtatgcggcg accgagttgc tcttgcccgg cgtcaatacg ggataatacc 9417
gcgccacata gcagaacttt aaaagtgctc atcattggaa aacgttcttc ggggcgaaaa 9477
ctctcaagga tcttaccgct gttgagatcc agttcgatgt aacccactcg tgcacccaac 9537
tgatcttcag catcttttac tttcaccagc gtttctgggt gagcaaaaac aggaaggcaa 9597
aatgccgcaa aaaagggaat aagggcgaca cggaaatgtt gaatactcat actcttcctt 9657
tttcaatatt attgaagcat ttatcagggt tattgtctca tgagcggata catatttgaa 9717
tgtatttaga aaaataaaca aataggggtt ccgcgcacat ttccccgaaa agtgccacct 9777
gaaattgtaa acgttaatat tttgttaaaa ttcgcgttaa atttttgtta aatcagctca 9837
ttttttaacc aataggccga aatcggcaaa atcccttata aatcaaaaga atagaccgag 9897
atagggttga gtgttgttcc agtttggaac aagagtccac tattaaagaa cgtggactcc 9957
aacgtcaaag ggcgaaaaac cgtctatcag ggcgatggcc cactacgtga accatcaccc 10017
taatcaagtt ttttggggtc gaggtgccgt aaagcactaa atcggaaccc taaagggagc 10077

ccccgattta gagcttgacg gggaaagccg gcgaacgtgg cgagaaagga agggaagaaa 10137

gcgaaaggag cgggcgctag ggcgctggca agtgtagcgg tcacgctgcg cgtaaccacc 10197

acacccgccg cgcttaatgc gccgctacag ggcgcgtccc attcgccatt caggctgcaa 10257

ataagcgttg atattcagtc aattacaaac attaataacg aagagatgac agaaaaattt 10317

tcattctgtg acagagaaaa agtagccgaa gatgacggtt tgtcacatgg agttggcagg 10377

atgtttgatt aaaaacataa caggaagaaa aatgccccgc tgtgggcgga caaaatagtt 10437

gggaactggg aggggtggaa atggagtttt taaggattat ttagggaaga gtgacaaaat 10497

agatgggaac tgggtgtagc gtcgtaagct aatacgaaaa ttaaaaatga caaaatagtt 10557

tggaactaga tttcacttat ctggttcgga tctcctaggc tcaagcagtg atcagatcca 10617

gacatgataa gatacattga tgagtttgga caaaccacaa ctagaatgca gtgaaaaaaa 10677

tgctttattt gtgaaatttg tgatgctatt gctttatttg taaccattat aagctgcaat 10737

aaacaagtta acaacaacaa ttgcattcat tttatgtttc aggttcaggg ggaggtgtgg 10797

gaggtttttt aaagcaagta aaacctctac aaatgtggta tggctgatta tgatcctcta 10857

gtacttctcg acaagcttgt cgagactgca ggctctagat tcgaaagcgg cc 10909

<210> 2
<211> 879
<212> PRT
<213> Artificial sequence

<220>
<223> IBDV VP1 protein

<400> 2
Met Ser Asp Val Phe Asn Ser Pro Gln Ala Arg Ser Thr Ile Ser Ala
1               5                   10                  15

Ala Phe Gly Ile Lys Pro Thr Ala Gly Gln Asp Val Glu Glu Leu Leu
            20                  25                  30

Ile Pro Lys Val Trp Val Pro Pro Glu Asp Pro Leu Ala Ser Pro Ser
        35                  40                  45

Arg Leu Ala Lys Phe Leu Arg Glu Asn Gly Tyr Lys Val Leu Gln Pro
    50                  55                  60

Arg Ser Leu Pro Glu Asn Glu Glu Tyr Glu Thr Asp Gln Ile Leu Pro
65                  70                  75                  80

Asp Leu Ala Trp Met Arg Gln Ile Glu Gly Ala Val Leu Lys Pro Thr
                85                  90                  95

Leu Ser Leu Pro Ile Gly Asp Gln Glu Tyr Phe Pro Lys Tyr Tyr Pro
            100                 105                 110

Thr His Arg Pro Ser Lys Glu Lys Pro Asn Ala Tyr Pro Pro Asp Ile
        115                 120                 125

Ala Leu Leu Lys Gln Met Ile Tyr Leu Phe Leu Gln Val Pro Glu Ala
    130                 135                 140

Asn Glu Gly Leu Lys Asp Glu Val Thr Leu Leu Thr Gln Asn Ile Arg
145                 150                 155                 160

Asp Lys Ala Tyr Gly Ser Gly Thr Tyr Met Gly Gln Ala Asn Arg Leu
                165                 170                 175

Val Ala Met Lys Glu Val Ala Thr Gly Arg Asn Pro Asn Lys Asp Pro
            180                 185                 190

Leu Lys Leu Gly Tyr Thr Phe Glu Ser Ile Ala Gln Leu Leu Asp Ile
        195                 200                 205

Thr Leu Pro Val Gly Pro Pro Gly Glu Asp Asp Lys Pro Trp Val Pro
    210                 215                 220

Leu Thr Arg Val Pro Ser Arg Met Leu Val Leu Thr Gly Asp Val Asp
225                 230                 235                 240

Gly Asp Phe Glu Val Glu Asp Tyr Leu Pro Lys Ile Asn Leu Lys Ser
                245                 250                 255

Ser Ser Gly Leu Pro Tyr Val Gly Arg Thr Lys Gly Glu Thr Ile Gly
            260                 265                 270

Glu Met Ile Ala Ile Ser Asn Gln Phe Leu Arg Glu Leu Ser Thr Leu
        275                 280                 285

Leu Lys Gln Gly Ala Gly Thr Lys Gly Ser Asn Lys Lys Lys Leu Leu
    290                 295                 300

Ser Met Leu Ser Asp Tyr Trp Tyr Leu Ser Cys Gly Leu Leu Phe Pro
305                 310                 315                 320

Lys Ala Glu Arg Tyr Asp Lys Ser Thr Trp Leu Thr Lys Thr Arg Asn
                325                 330                 335

Ile Trp Ser Ala Pro Ser Pro Thr His Leu Met Ile Ser Met Ile Thr
            340                 345                 350

Trp Pro Val Met Ser Asn Ser Pro Asn Asn Val Leu Asn Ile Glu Gly
        355                 360                 365

Cys Pro Ser Leu Tyr Lys Phe Asn Pro Phe Arg Gly Gly Leu Asn Arg
    370                 375                 380

Ile Val Glu Trp Ile Leu Ala Pro Glu Glu Pro Lys Ala Leu Val Tyr
385                 390                 395                 400

Ala Asp Asn Ile Tyr Ile Val His Ser Asn Thr Trp Tyr Ser Ile Asp
                405                 410                 415

Leu Glu Lys Gly Glu Ala Asn Cys Thr Arg Gln His Met Gln Ala Ala
            420                 425                 430

Met Tyr Tyr Ile Leu Thr Arg Gly Trp Ser Asp Asn Gly Asp Pro Met
        435                 440                 445

Phe Asn Gln Thr Trp Ala Thr Phe Ala Met Asn Ile Ala Pro Ala Leu
    450                 455                 460

Val Val Asp Ser Ser Cys Leu Ile Met Asn Leu Gln Ile Lys Thr Tyr
465                 470                 475                 480

Gly Gln Gly Ser Gly Asn Ala Ala Thr Phe Ile Asn Asn His Leu Leu
                485                 490                 495

Ser Thr Leu Val Leu Asp Gln Trp Asn Leu Met Arg Gln Pro Arg Pro
            500                 505                 510

Asp Ser Glu Glu Phe Lys Ser Ile Glu Asp Lys Leu Gly Ile Asn Phe
        515                 520                 525

Lys Ile Glu Arg Ser Ile Asp Asp Ile Arg Gly Lys Leu Arg Gln Leu
    530                 535                 540

Val Leu Leu Ala Gln Pro Gly Tyr Leu Ser Gly Gly Val Glu Pro Glu
545                 550                 555                 560

Gln Ser Ser Pro Thr Val Glu Leu Asp Leu Leu Gly Trp Ser Ala Thr
                565                 570                 575

Tyr Ser Lys Asp Leu Gly Ile Tyr Val Pro Val Leu Asp Lys Glu Arg
            580                 585                 590

Leu Phe Cys Ser Ala Ala Tyr Pro Lys Gly Val Glu Asn Lys Ser Leu
        595                 600                 605

Lys Ser Lys Val Gly Ile Glu Gln Ala Tyr Lys Val Val Arg Tyr Glu
    610                 615                 620

Ala Leu Arg Leu Val Gly Gly Trp Asn Tyr Pro Leu Leu Asn Lys Ala
625                 630                 635                 640

Cys Lys Asn Asn Ala Gly Ala Ala Arg Arg His Leu Glu Ala Lys Gly
                645                 650                 655

Phe Pro Leu Asp Glu Phe Leu Ala Glu Trp Ser Glu Leu Ser Glu Phe
            660                 665                 670

Gly Glu Ala Phe Glu Gly Phe Asn Ile Lys Leu Thr Val Thr Ser Glu
        675                 680                 685

Ser Leu Ala Glu Leu Asn Lys Pro Val Pro Pro Lys Pro Pro Asn Val
    690                 695                 700

Asn Arg Pro Val Asn Thr Gly Gly Leu Lys Ala Val Ser Asn Ala Leu
705                 710                 715                 720

Lys Thr Gly Arg Tyr Arg Asn Glu Ala Gly Leu Ser Gly Leu Val Leu
                725                 730                 735

Leu Ala Thr Ala Arg Ser Arg Leu Gln Asp Ala Val Lys Ala Lys Ala
            740                 745                 750

Glu Ala Glu Lys Leu His Lys Ser Lys Pro Asp Asp Pro Asp Ala Asp
      755                 760                 765

Trp Phe Glu Arg Ser Glu Thr Leu Ser Asp Leu Leu Glu Lys Ala Asp
    770                 775                 780

Ile Ala Ser Lys Val Ala His Ser Ala Leu Val Glu Thr Ser Asp Ala
785                 790                 795                 800

Leu Glu Ala Val Gln Ser Thr Ser Val Tyr Thr Pro Lys Tyr Pro Glu
                805                 810                 815

Val Lys Asn Pro Gln Thr Ala Ser Asn Pro Val Val Gly Leu His Leu
            820                 825                 830

Pro Ala Lys Arg Ala Thr Gly Val Gln Ala Ala Leu Leu Gly Ala Gly
      835                 840                 845

Thr Ser Arg Pro Met Gly Met Glu Ala Pro Thr Arg Ser Lys Asn Ala
    850                 855                 860

Val Lys Met Ala Lys Arg Arg Gln Arg Gln Lys Glu Ser Arg Gln
865                 870                 875

<210>  3
<211>  13
<212>  PRT
<213>  Infectious bursal disease virus (IBDV)

<400>  3

Gly Arg Trp Ile Arg Thr Val Ser Asp Glu Asp Leu Glu
1               5                   10

<210> 4
<211> 37
<212> DNA
<213> Artificial sequence

<223> 5' primer oligonucleotide used for generating the different
      deletion mutants of the terminal carboxy end of His-VP3 in
      combination with SEQ. ID. NO: 5, SEQ. ID. NO: 6, SEQ. ID. NO: 7,
      SEQ. ID. NO: 8 and SEQ. ID. NO: 9, respectively

<400> 4
gggggaattc atggcatcag agttcaaaga gacccc                          37

<210> 5
<211> 31
<212> DNA
<213> Artificial sequence

<223> 3' primer oligonucleotide used for generating His-VP3Δ248-257
      mutant in combination with SEQ. ID. NO: 4

<400> 5
cgcgggtacc ttaccagcgg cccagccgac c                                31

<210> 6
<211> 33
<212> DNA
<213> Artificial sequence

<223> 3' primer oligonucleotide used for generating His-VP3Δ243-257
      mutant in combination with SEQ. ID. NO: 4

<400> 6
cgcgggtacc ttaaccaggg ggtctctgtg ttg                              33

<210> 7
<211> 33
<212> DNA
<213> Artificial sequence

<223> 3' primer oligonucleotide used for generating His-VP3Δ238-257
      mutant in combination with SEQ. ID. NO: 4

<400> 7
cgcgggtacc ttatgttgga gcattgggtt ttg                              33

<210> 8
<211> 31
<212> DNA
<213> Artificial sequence

<223> 3' primer oligonucleotide used for generating His-VP3Δ233-257
      mutant in combination with SEQ. ID. NO: 4

<400> 8
cgcgggtacc ttattttggc ttgggctttg g                                31

<210> 9
<211> 31
<212> DNA
<213> Artificial sequence

<223> 3' primer oligonucleotide used for generating His-VP3Δ228-257
      mutant in combination with SEQ. ID. NO: 4

<400> 9
cgcgggtacc ttatggtaga gcccgcctgg g                                31